# EUROPÄISCHE PATENTANMELDUNG

(11) **EP 2 275 802 A1**
(43) Veröffentlichungstag der Anmeldung: **19.01.2011**
(21) Anmeldenummer: 10010982.6
(22) Anmeldetag: 09.08.2001
(51) Int. Cl.: G01N 21/64, G01N 21/77, G01N 33/543

(54) **Wellenleitergitterstruktur und optische Messanordnung**

(30) Priorität: 09.08.2000 CH 15592000
(62) Teilanmeldung aus: 10001618.7
(71) Anmelder: Artificial Sensing Instruments ASI AG, 8052 Zürich (CH)
(72) Erfinder: Tiefenthaler, Kurt, 8046 Zürich (CH)
(74) Vertreter: Frei Patent Attorneys

(57) **Zusammenfassung**

Die vorliegende Erfindung beschreibt (bio)chemofunktionale Wellenleitergitterstrukturen bestehend aus mindestens einer (bio)chemofunktionalen Wellenleitergitterstruktureinheit oder mindestens einer (bio)chemofunktionalen Sensorstelle mit Lichtstrahlseparation zulassenden Strahlführungen sowie markierungsfreie als auch auf Markierung beruhende Detektionsmethoden für die Parallelanalyse.

## Beschreibung

In den PCT-Anmeldungen PCT/EP94/02361 und PCT/CH98/00389 sind bereits verschiedene Wellenleitergitterstrukturen beschrieben. Die Strahlführung ist jedoch nicht optimal. Der reflektierte Strahl einer ersten einfallenden Lichtwelle koinzidiert mit der Richtung einer zweiten einfallenden Lichtwelle. Der reflektierte Lichtstrahl und der zweite einfallende Lichtstrahl haben entgegengesetzt gleiche Richtung, was zu Störungen führen kann. Die PCT-Anmeldungen PCT/EP94/02361 und PCT/CH98/00389 zeigen nicht, wie eine Wellenleitergittersensorstruktur, die aus zweidimensional angeordneten Sensorstellen besteht, im Fall der markierungsfreien Detektion parallel ausgelesen werden kann. Parallele Auslesung ist jedoch notwendig, um einen hohen Durchsatz zu erreichen.

In der europäischen Patentanmeldung EP 0 482 377 A2 wird das Wellenleitergitter mit einem fokussierenden Lichtfeld beleuchtet. Ein fokussierendes Lichtfeld eignet sich nicht für die gleichzeitige Beleuchtung eines zweidimensionalen Arrays von Wellenleitergittersensoren. Zudem ist in dieser Anmeldung keine Methode zur Temperaturkompensation erwähnt.

In Sensors and Actuators B 38-39 (1997), 116-121 werden eine Sensorstelle und eine Referenzstelle des Wellenleitergitters im Multiplexverfahren beleuchtet. Es ist auch keine eindimensionale oder zweidimensionale Strahlaufweitung des einfallenden Lichtstrahls gezeigt.

Die vorliegende Erfindung löst die Aufgabe, einen optischen Sensor zu schaffen, welcher
(1) mehrere eindimensional oder zweidimensional angeordnete Sensorstellen basierend auf Wellenleitergitterstrukturen via geeigneter Strähläufweitungsoptik gleichzeitig beleuchtet
(2) Separation der Lichtfelder bzw. der Lichtstrahlen gewährleistet
(3) Lichtfelder auf einem Detektor bzw. Detektorarray (z.B. Pixelarraydetektor) erzeugt, die sich auf dem Detektor bzw. Detektorarray nicht überlagern
(4) Messignale über nichtreflektierte Lichtfelder generiert
(5) Messignale, die eine geringe Temperaturabhängigkeit aufweisen, generiert
(6) Messungen in einem Scanmodusverfahren ohne bewegliche Mechanik mit grossem dynamischen Bereich durchführt
(7) zur Bestimmung der Resonanzstelle die Scanverteilung (d.h. die in einem bestimmten Detektorbereich gemessene Lichtintensität in Funktion des Scanparameters und/oder die in einem bestimmten Detektorbereich gemessene Lichtintensität in Funktion der Detektorkoordinaten) mit einem Schwerpunktsverfahren oder mit einem Datenfit eines Teils der Scanverteilung (Bereich des Maximums, Bereich des maximalen Anstiegs, Bereich des konstanten Anstiegs) auswertet
(8) referenzierte Sensorsignale durch Auswertung eines Signalpfads und zumindest eines Referenzpfads generiert
(9) die Messgenauigkeit,mittels Scanmittelungsverfahren oder Resonanzstellenmittelungsverfahren erhöht
(10) die Auswertung von Microplates, Microarrays und Lab-on-chips erlaubt.

Die Erfindung schlägt auch Wellenleitergitterstruktureinheiten vor, die eine Separierung der einfallenden, reflektierten und gebeugten Lichtstrahlen erlauben, ohne den Strahlengang gegenüber der Einfallsebene verkippen zu müssen. Ferner kann die Auslesung der vier ausgekoppelten Moden TE+, TE-, TM+, TM- (Notation: TE+: transversal elektrischer Mode in (+x)-Richtung (Vorwärtsrichtung), TE-: transversal elektrischer Mode in Rückwärtsrichtung, TM+: transversal magnetischer Mode in Vorwärtsrichtung, TM-: transversal magnetischer Mode in Rückwärtsrichtung) auf einem einzigen eindimensionalen oder zweidimensionalen positionsempfindlichen Detektor (z.B. Pixelarraydetektor) erfolgen, wobei sich vorzugsweise zwischen Detektor und Wellenleitergitterstruktur eine Linsenoptik befindet. Werden eindimensionale oder zweidimensionale Arrays von Wellenleitergitterstruktureinheiten (bzw. Arrays von Sensorstellen) verwendet, wie sie z.B. bei Microplates oder Microarrays zum Einsatz kommen, empfiehlt es sich, mit einem zweidimensionalen Pixelarraydetektor zu arbeiten, da alle ausgekoppelten und/oder abgestrahlten Lichtwellen auf die zweidimensionale Detektionsfläche fallen können. Die Arrays aus Wellenleitergitterstruktureinheiten (bzw. Arrays aus Sensorstellen) können kartesisch, matrixförmig oder kreis(ring)förmig auf einer runden oder (recht)eckigen Scheibe (Platte) angeordnet sein.
Figur 1 zeigt eine Dreigitteranordnung mit koinzidierender Strahlführung.
Figuren 2a, 2b, 2c zeigen Wellenleitergitterstruktureinheiten, welche auch arrayförmig angeordnet werden können.
Figuren 3a, 3b, 4a, 4b, 5a, 5b zeigen verschiedene Messanordnungen.
Figuren 6 und 7 zeigen einen Array von Wellenleitergitterstruktureinheiten (i,j) bestehend aus einem Signalpfad S(i,j) und einem Referenzpfad R(i,j).
Figur 8 zeigt einen Array von Sensoreinheiten (i,j) bestehend aus einem Signalpfad S(i,j) und einem Referenzpfad R(i,j) auf einem grossen Wellenleitergitter.
Figur 9 zeigt die Sensitivität dN/dnC eines Wellenleiters bezüglich einer Brechzahländerung dnC des Covermediums C in Funktion der Dicke dF des wellenleitenden Films F.
Figur 10 zeigt die Sensitivität dN/d(dA) eines Wellenleiters bezüglich einer Schichtdickenänderung d(dA) der chemosensitiven Schicht A (A=adlayer) in Funktion der Dicke dF des wellenleitenden Films F.
Figur 11 zeigt einen Messaufbau in schematischer Weise, bei dem das Detektionssytem nicht im reflektierten Strahlengang steht.

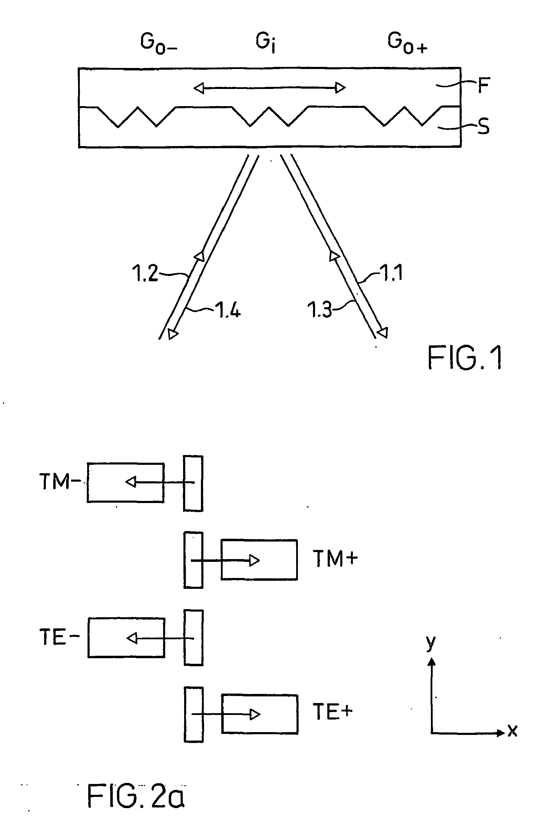

Figur 1 zeigt eine Dreigitteranordnung mit koinzidierender Strahlführung. Der reflektierte Strahl 1.1 der linken einfallenden Lichtwelle 1.2 läuft der rechten einfallenden Lichtwelle 1.3 (mit Reflexionsstrahl 1.4) direkt entgegen. Das mittlere Gitter ist das Einkopplungsgitter Gᵢ. Rechts und links davon befinden sich die beiden Auskopplungsgitter Gₒ₊ und Gₒ₋. Der wellenleitende Film F befindet sich auf dem Substrat S. Die Pfeile im wellenleitenden Film F stellen die Ausbreitungsrichtung der Moden dar. Bei senkrechtem Lichteinfall läuft gar der reflektierte Lichtstrahl in sich zurück. Ist speziell die Lichtquelle eine Laserquelle (Laserdiodenquelle), so kann eine koinzierende Strahlführung Laserfluktuationen verursachen, da zum eigentlichen Laserresonator ein externer Resonator dazugeschaltet wird. Laserfluktationen verursachen Instabilitäten im Messignal.

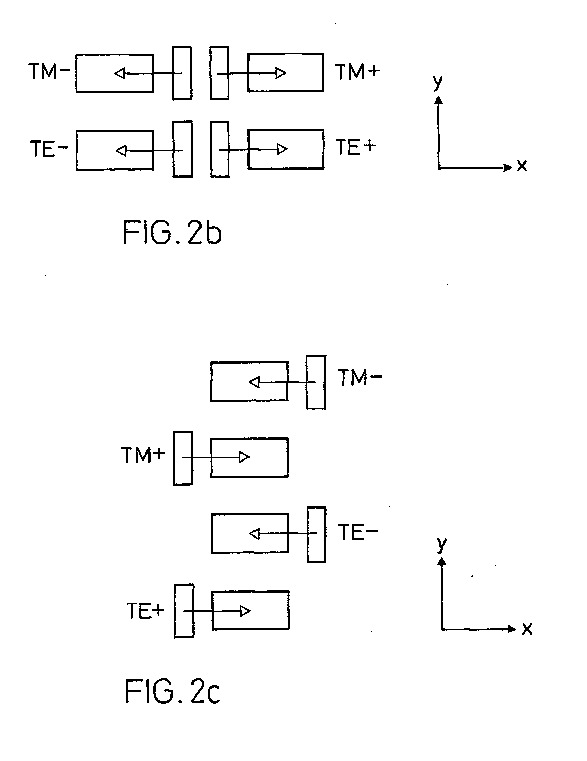

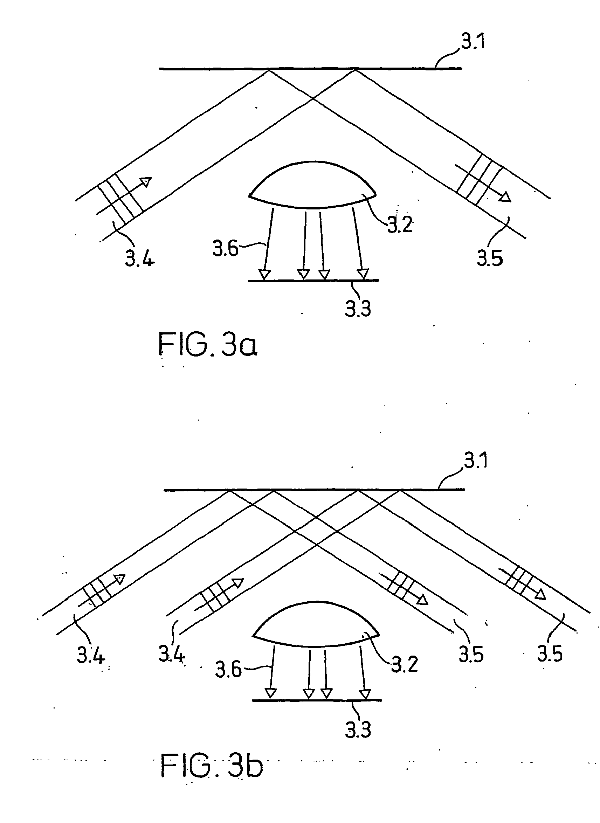

Figuren 2a, 2b, 2c zeigen spezielle Wellenleitergitterstruktureinheiten einer Wellenieftergitterstruktur, wobei die kleinen Rechtecke Einkopplungsgitter und die grossen Rechtecke Auskopplungsgitter bedeuten. Die Gitterstriche sind parallel zu y-Achse. Die Gitterperioden sind jeweils so gewählt, dass Modenanregung und Modenauskopplung mit den vorgeschlagenen Messanordnungen (siehe Figuren 3,4 und 5) möglich sind. In den Figuren 2a bis 2c kann sich nach dem Auskopplungsgitter jeweils auch noch ein Stopp-Gitter (oder ein Stopp-Taper oder eine Stopp-Rille) befinden. Das Stopp-Gitter beugt das verbleibende Modenlicht in eine für die Messung nicht relevante Richtung. Figur 2c stellt eine kompakte Form einer Wellenleitergitterstruktureinheit dar, da die Ausdehnung der Wellenleitergitterstruktureinheit in x-Richtung kleiner ist als in Figur 2a und Figur 2b. Es existieren natürlich auch symmetrische Varianten von den Figuren 2a, 2b und 2c. Beispielsweise können die Figuren um 180 Grad gedreht werden. Die Einkopplungsgitter können stärker moduliert sein als die Auskopplungsgitter. Die anzuregenden Moden TE+, TE-, TM+ und TM- mit vorzugsweiser Modenzahl m=0 sind als Pfeile eingezeichnet und bezeichnet. Die Reihenfolge der vier 'sensing pads', die jeweils aus einem Einkopplungsgitter und einem Auskopplungsgitter bestehen, wie auch die Reihenfolge der anzuregenden Moden kann ohne Einschränkung der Allgemeinheit vertauscht werden. Die 'sensing pads' können als planare Wellenleiter oder als Streifenwellenleiter ausgelegt sein.

In den Figuren 2a, 2b, 2c besteht jeweils eine Wellenleitergitterstruktureinheit aus vier 'sensing pads'. Jeder 'sensing pad' ist einem der vier Moden zugeordnet. Es können jedoch auch je zwei 'sensing pads' (gegebenenfalls auch alle vier 'sensing pads') überlagert werden. Dadurch kommen bidiffraktive bzw. multidiffraktive Gitter zum Einsatz. Die Ueberlagerung von je zwei 'sensing pads' wird anhand von Figur 2 erklärt: Der für den Mode TE- zuständige 'sensing pad' wird mit dem für den Mode TMzuständigen 'sensing pad' überlagert. Der für den Mode TE+ zuständige 'sensing pad' wird mit dem für den Mode TM+ zuständigen 'sensing pad' überlagert. In den Figuren 2 kann auch nur ein 'sensing pad' für einen Mode X=TE+(TM+) in (+x)-Richtung und ein 'sensing pad' für einen Mode X=TE-(TM-) in (-x)-Richtung vorhanden sein.

Die Wellenleiterstruktur kann aus einem (Array von) Streifenwellenleitern und/oder aus einem wellenleitenden Film (aus z.B. TiO₂, Ta₂O₅, ZrO₂, Si₃N₄ etc.) und/oder aus einem Schichtsystem aus mehreren Schichten bestehen, wobei gewährleistet sein muss, dass das Schichtsystem wellenleitende Eigenschaften aufweist. Es muss also die effektive Brechzahl N der geführten Lichtwelle (mit Modenzahl m=0,1,2_{,}...) grösser als die Brechzahl des Substrats und grösser als die Brechzahl des Covermediums sein. Vorzugsweise ist im Schichtsystem eine Schicht mit hoher Brechzahl vorhanden. Die hohe Brechzahl garantiert eine hohe elektromagnetische Feldstärke an der Oberfläche des Wellenleiters und damit eine hohe Sensitivität bei der Detektion von Oberflächenphänomenen (Adsorption, selektive Bindungsereignisse, Oberflächenreaktionen, holistische Zellereignisse etc.). Es können auch absorbierende Wellenleiterstrukturen zum Einsatz kommen. Das Substrat kann aus einem durchsichtigen Material (Glas oder Kunststoff) bestehen.

Die Herstellung von Wellenleitergitterstrukturen und die dabei zum Einsatz kommenden Materialien sind in den PCT-Anmeldungen PCT/EP94/02361 und PCT/CH98/00389 beschrieben. Die in den PCT-Anmeldungen PCT/EP94/02361 und PCT/CH98/00389 beschriebenen Wellenleitergitterstrukturanordnungen können ebenfalls um Stopp-Gitter oder Stopp-Taper oder Stopp-Rillen ergänzt werden. Alle Wellenleitergitterstrukturen - auch jene aus PCT/EP94/02361 und PCT/CH98/00389 können mit einer Lichtwelle beleuchtet werden, deren Wellenvektorkomponente parallel zu den Gitterlinien leicht von null verschieden ist.

Die Gitter können Rechteckgitter, Sinus(Kosinus)gitter, Sägezahngitter etc. sein. Die Gitter können nass oder trocken geätzt werden oder mit einem Replikationsverfahren ((Heiss)-Prägetechnik, Spritzgussverfahren (mit oder ohne Kompressionschritt(e)), etc) hergestellt werden. Die Masken können photolithographisch, gegebenenfalls mit Einsatz eines Elektronenstrahls, hergestellt werden. Masken braucht es für die Herstellung der Gitter bzw. der Abformungsvorlage. Das Gitter kann sich an einem Interface der Wellenleiterstruktur (d.h. am Interface einer Schicht der Wellenleiterstrukur) oder in der Wellenleiterstruktur befinden. Die Gitter können auch fokussierend sein.

Eine Wellenleitergitterstruktureinheit wird chemosensitiv (chemofunktional) gemacht, indem eine chemosensitive Substanz zumindest in der Region der Sensorgitter (zumindest teilweise) auf (oder in) die Wellenleitergitterstruktureinheit aufgebracht (oder eingebracht) wird. Im Auskopplungswinkel-Scanmodus (siehe weiter unten) entsprechen die Auskopplungsgitter den Sensorgittern, im Einfallswinkel-Scanmodus (siehe weiter unten) oder im Wellenlängen-Scanmodus (siehe weiter unten) entsprechen die Einkopplungsgitter den Sensorgittem. Binden (Bio)Moleküle an die (bio)chemosensitive Substanz, so ändert sich die (eventuell komplexe) effektive Brechzahl einer geführten Lichtwelle.

Da das Sensorgitter auf der Wellenleitergitterstruktureinheit manchmal lokal beschränkt ist, ist es vorteilhaft, wenn die chemofunktionale Schicht das komplette Sensorgitter abdeckt. Der Rand der chemofunktionalen Schicht verläuft ausserhalb des Sensorgitters. Auch ist es vorteilhaft, wenn das komplette Sensorgitter (mit einer ebenen Welle und/oder einer geführten Welle) und die das Sensorgitter umgebende nähere Umgebung beleuchtet wird. Ein kleines Beam Stirring bleibt damit ohne Wirkung (zumindest im Fall, wo mit einem Signalpfad und einem Referenzpfad gearbeitet wird), da dann selbst mit Beam Stirring immer noch das komplette Sensorgitter beleuchtet bleibt.

Als (bio)chemofunktionale Schichten können neben (bio)molekularen Bindungspartnern (wie z.B. Antikörper, Antigene, Rezeptoren, Peptide, Phagen, 'single stranded' DNA(RNA)-Abschnitte, Gene, Genabschnitte, Targets, Proteine, Bindungsproteine, Enzyme, Inhibitoren, Nukleinsäuren, Nukleotide, Oligonukleotide, SNP, Allergene, Pathogene, Kohlenhydrate, Metaboliten, Hormone etc) auch 'molecular imprinted polymers' (wie z.B. Plastikantikörper, Plastikantigene etc.) oder (lebende) Zellen zum Einsatz kommen. Die Bindungsvorgänge (bzw. die (bio)chemischen Reaktionen) können auch hier an der Oberfläche, im Volumen oder sowohl an der Oberfläche als auch im Volumen der (bio)chemofunktionalen Schicht erfolgen.

Eine Wellenleitergitterstruktureinheit kann auch zur Referenzierung eingesetzt werden. Das Sensorsignal S der Signal-Wellenleitergitterstruktureinheit kann mit dem Sensorsignal R der Referenz-Wellenleitergitterstruktureinheit referenziert werden. Die chemofunktionale Signalschicht belegt (zumindest teilweise) die Signal-Wellenleitergitterstruktureinheit (Signalpfad) und die chemofunktionale Referenzschicht (zumindest teilweise) die Referenz-Wellenleitergitterstruktureinheit (Referenzpfad). Das referenzierte-Sensorsignal S_{ref} ist dann S_{ref} = S - R. Die Berandung der chemofunktionalen Schichten kann auch ausserhalb der Gitter liegen. Eine chemofunktionale Schicht kann auch zumindest die gesamte Wellenleitergitterstruktureinheit bedecken.

Eine für die Referenzierung spezielle Wellenleitergitterstruktureinheit kann man sich auch wie folgt vorstellen: Bei zwei (benachbarten) Wellenleitergitterstruktureinheiten ist jeweils nur ein 'sensing pad' vorhanden. Die anderen 'sensing pads' werden über Modulationsstärke = 0 zum Verschwinden gebracht, sind also nicht vorhanden. Die so generierten Wellenleitergitterstruktureinheiten sind aber als zu den Figuren 2a,2b,2c zugehörig zu betrachten, indem die Modulationsstärke (=0) als Gittereigenschaft aufgefasst wird. Die beiden (in beliebigem Abstand nebeneinander liegenden) sensing pads (mit jeweils einem Einkopplungsgitter und einem Auskopplungsgitter oder gegebenenfalls jeweils nur mit einem Einkopplungsgitter) der beiden Wellenleitergitterstruktureinheiten mit vorzugsweise gleichem Mode tragen unterschiedliche chemofunktionale Schichten, wie in der Patentanmeldung WO 99/13320 beschrieben. Durch Referenzierung der beiden Messignale (Sensorsignal und Referenzsignal) z.B. durch Differenzbildung der beiden Auskopplungswinkel im Auskopplungswinkel-Scanmodus oder durch Differenzbildung der beiden Resonanzwellenlängen für Gittereinkopplung im Wellenlängen-Scanmodus (siehe weiter unten) oder durch Differenzbildung der beiden Resonanzeinfallswinkel für Gittereinkopplung im Einfallswinkel-Scanmodus (siehe weiter unten) kann die auf beiden sensing pads vorhandene Drift (wie Temperaturdrift, thermo-optische Drift, photochemische Drift, Wellenlängendrift, Einfallswinkeldrift etc.) eliminiert werden. Die wellenlängenabhängige Sensitivität kann bei der Referenzierung mitberücksichtigt werden. Bei Referenzierung mit Moden unterschiedlicher Polarisation und/oder Modenzahl muss die unterschiedliche Sensitivität der Polarisation und/oder Modenzahl berücksichtigt werden.

Die chemofunktionale Referenzschicht soll die gleiche oder nahezu die gleiche nicht spezifische Bindung (NSB) aufweisen wie die chemosensitive Signalschicht oder keine oder beinahe keine NSB aufweisen. Die chemofunktionale Referenzschicht kann durchaus auch spezifische Bindungspartner enthalten, solange man durch Vorinformation weiss, dass kein Analyt der Probe an diese Bindungspartner binden kann. Bei Vergleichsstudien kann man sich auch vorstellen, dass der (oder ein) Analyt der Probe durchaus an die spezifischen Bindungspartner der chemofunktionalen Referenzschicht bindet.

Die chemofunktionalen Schichten können auch (lebende) Zellen (Human-, Tier-Pflanzenzellen etc.) gegebenenfalls auf modifizierten Oberflächen (Silanschichten, Polymerschichten, Lipidschichten (mit oder ohne Ionenkanäle, Monolayers, Bilayers etc.) oder biokompatiblen Oberflächen sein. Der Referenzpfad enthält - falls vorhanden - andere (oder modifizierte) Zellen. Die (flüssige) Probe für Signal- und Referenzpfad können gleich oder unterschiedlich sein. Anstelle von biomolekularen Interaktionen wird hier das Zellverhalten holistisch (als Ganzes) durch Messung von (komplexen) effektiven Brechzahländerungen (und davon abgeleiteten Grössen) an Zellen oder von Aenderungen von Fluoreszenz(Lumineszenz)Signalen an (fluoreszenzmarkierten oder lumineszenzmarkierten) Zellen studiert. Die Zellbelegungsdichte der beiden Pfade soll (eventuell bis auf den Rauschabstand des referenzierten Messignals) gleich oder beinahe gleich sein.

Es können in einer Wellenleitergitterstruktureinheit auch die sensing pads für den einen Mode (in Vorwärts- und Rückwärtsrichtung) (z.B. TE+,TE-) als Referenzpfad für die sensing pads mit dem anderen Mode (in Vorwärts- und Rückwärtsrichtung) (z.B. TM+,TM-) (=Signalpfad) (oder auch umgekehrt) angesehen werden. Bedeckt eine chemofunktionale Schicht beide Pfade, so sind chemofunktionale Signalschicht und chemofunktionale Referenzschicht identisch (also auch gleich dick).

Figuren 3a, 3b, 4a, 4b, 5a, 5b zeigen verschiedene Messanordnungen (die Lichtwellen sind mit Pfeilen eingezeichnet), wobei jeweils zumindest die Einkopplungsgitter beleuchtet werden. Es sind 3.1 eine Wellenleitergitterstruktur, 3.2 eine Linse (ein Linsensytem), 3.3 ein Detektor, 3.4 einfallende Lichtstrahlen, 3.5 reflektierte Lichtstrahlen, 3.6 auf den Detektor auftreffende Lichtstrahlen. Die Beleuchtung kann mit einem (aufgeweiteten) Lichtstrahl oder einem Lichtband erfolgen. Das Lichtband (die Lichbänder) erlaubt (erlauben) die simultane Beleuchtung von mehreren in Lichtbandrichtung (senkrecht zur Zeichenebene) angeordneten Wellenleitergitterstruktureinheiten. Ein zweidimensional aufgeweiteter Lichtstrahl erlaubt die simultane Beleuchtung von zweidimensionalen Arrays von Wellenleitergitterstruktureinheiten. Falls die Einkopplungsgitter zwei unterschiedliche x-Koordinaten auf der x-Achse haben (siehe Figuren 2b und 2c), können die beiden einfallenden Lichtwellen zwei unterschiedliche Stellen der Wellenleitergitterstruktureinheit treffen (siehe Figuren 3b, 4b, 5b). Diese zwei unterschiedlichen Stellen beinhalten jeweils Einkopplungsgitter für Modenanregung in (+x)-Richtung bzw. für Modenanregung in (-x)-Richtung (siehe Figuren 2b und 2c). Die einfallenden (bzw. reflektierten) Lichtwellen können ebene Wellen (dargestellt als Pfeile mit drei dazu senkrechten Strichen) oder (leicht) konvergierende (bzw. divergierende) Wellen sein.

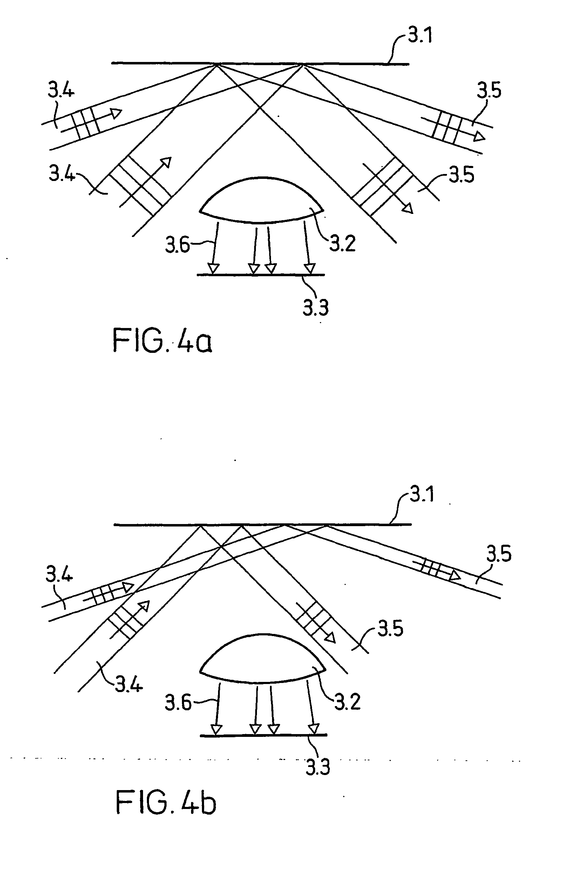

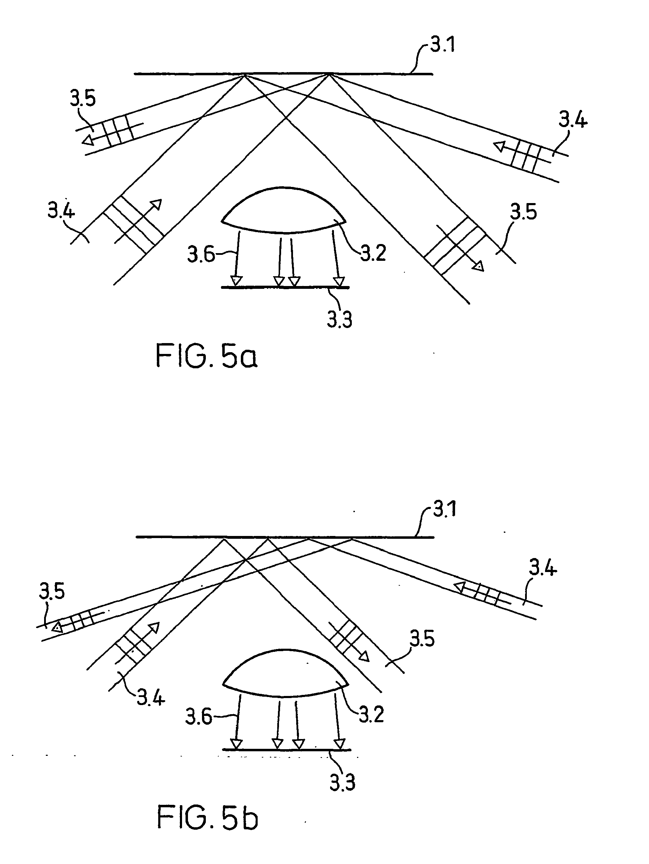

In den Figuren 3b, 4a, 4b beleuchten die beiden einfallenden Lichtwellen von links unten her die Wellenleitergitterstruktur. In Figur 3b sind die beiden Einfallswinkel gleich, in den Figuren 4a und 4b sind die beiden Einfallswinkel verschieden. In den Figuren 5a und 5b beleuchtet die eine einfallende Lichtwelle die Wellenleitergitterstruktur von links unten her, die zweite einfallende Lichtwelle die Wellenleitergitterstruktur von rechts unten her, wobei die beiden Einfallswinkel sich betragsmässig unterscheiden.

Die Figuren 3a, 3b, 4a, 4b, 5a, 5b haben gemeinsam, dass jeweils vier ausgekoppelte Lichtwellen existieren, die durch die Auskopplung der Moden TE+, TM+, TE-, TMentstehen. Die vier Lichtwellen werden mit einer Linse oder mit mehreren Linsen fokussiert, wobei die Fokusse der ausgekoppelten Lichtwellen nicht unbedingt auf der eindimensionalen bzw. zweidimensionalen Detektorfläche liegen müssen. Ein Lichtfleck auf einem Pixelarraydetektor soll durchaus eine gewisse Ausdehnung zeigen und nicht nur ein Pixel beleuchten. Die vier Strahlen sind jeweils schematisch als vier Pfeile eingezeichnet. Die Reihenfolge der Strahlen wird durch die Gitterperioden festgelegt. Mit einer (oder mehreren) Linse(n) bzw. mit einem Linsensystem können auch die Lichtwellen von mehreren Wellenleitergitterstruktureinheiten auf einen einzigen ein(zwei)dimensionalen Pixelarraydetektor gebracht werden.

Die in den PCT-Anmeldungen PCT/EP94/02361 und PCT/CH98/00389 beschriebenen Wellenleitergitterstrukturen (ein(zwei)dimensionale Arrays von Wellenleitergitterstruktureinheiten) können ebenfalls nur mit einem einzigen eindimensionalen oder zweidimensionalen Pixelarraydetektor ausgelesen werden, indem die ausgekoppelten bzw. abgestrahlten Lichtfelder durch geeignete Wahl der Gitterperioden bzw. des sich zwischen Wellenleiter und Detektor befindlichen Linsensystems auf einen Array von Detektoren fallen (siehe auch weiter unten).

Vorteilhaft ist es auch, wenn die Lichtstrahlen in Röhren, Stäben, Blöcken oder gebohrten Blöcken (Strahlengang ist in der Bohrung) einzeln oder gemeinsam geführt und dadurch geschützt werden. Als Material für die Stäbe und Blöcke eignet sich Glas, Zerodur, Kunststoffe sowie glasähnliche Materialien. Mit Hilfe eines geschützten Strahlengangs werden Störungen infolge Temperatur- und/oder Luftfluktuationen (Luftturbulenzen) reduziert und damit die Messgenauigkeit erhöht. Diese Schutzvorrichtungen können auch bei den Anordnungen, wie sie in PCT/EP94/02361 und PCT/CH98/00389 beschrieben werden, angewendet werden.

Die einfallenden Lichtstrahlen sind vorzugsweise unter 45 Grad linear polarisiert, sodass die Lichtstrahlen (bzw. Lichtbänder) sowohl TE-Moden als auch TM-Moden anregen können. Die einfallenden Lichtstrahlen können nur die Einkopplungsgitter oder aber auch die ganze Wellenleitergitterstruktur beleuchten. Die Gitterperioden der Einkopplungsgitter und die Einfallswinkel sind derart aufeinander abgestimmt, dass es jeweils zur Modenanregung kommt. Die Einkopplungsgitter sind derart ausgelegt, dass das Licht aus einem grösseren Winkelsegment eingekoppelt werden kann.

Auch wenn die Wellenleitergitterstruktur ein wenig bezüglich den einfallenden Lichtwellen in der (x,y)-Ebene verschoben wird, hat dies (fast) keinen Einfluss auf die Messung. da Lichtfleckverteilungen bzw. (relative) Lichtfleckpositionen bestimmt werden und diese nicht von der Intensität abhängen. Da es im Auskopplungswinkel-Scanmodus im wesentlichen auf die relative Lage der (vier) Lichtflecken auf dem Array von Detektoren darauf ankommt, hat eine leichte Verkippung des Sensor Chips bezüglich der Messanordnung (fast) keinen Einfluss auf die relative Lage der Lichtflecken und damit (fast) keinen Einfluss auf das Messergebnis.

In den Figuren 3a, 3b, 4a, 4b fallen eine oder zwei Lichtwellen von unten links her auf die Wellenleitergitterstruktur und erzeugen die Moden TE+, TE-, TM+, TM-. Durch geeignete Wahl der Gitterperiode ist es mit einer derart einfallenden Lichtwelle durchaus auch möglich, die Moden TE- und TM- zu erzeugen.

Zu den Figuren 3a, 3b, 4a, 4b, 5a, 5b bestehen auch folgende Alternativen: Die einfallenden Lichtstrahlen kommen beinahe senkrecht von unten, weisen also einen kleinen Einkopplungswinkel (bezogen auf die Senkrechte zur Wellenleiterebene) auf, dafür sind die Auskopplungswinkel wesentlich grösser als die Einkopplungswinkel. Die ausgekoppelten Lichtstrahlen fallen entweder schräg nach unten rechts oder schräg nach unten links oder teils schräg nach unten rechts und teils schräg nach unten links auf den (die) ein(zwei)dimensionalen Detektorarray(s) (z.B. Pixelarraydetektor(en)) (mit Fokussierungsoptik). Die ausgekoppelten Lichtwellen beleuchten wiederum verschiedene Stellen auf dem Detektorarray, was durch geeignete Wahl der Gitterperioden erreicht wird.

Die Position eines Lichtflecks (Positionen mehrerer Lichtflecke) lässt (lassen) sich nicht nur mit einem ein(zwei)dimensionalen Pixelarraydetektor (z.B. CCD-Array, Photodiodenarray oder CMOS-Array), sondern auch anders - z.B. mit einem (ein(zwei)dimensionalen Array von) positionssensitiven Detektor(en) (PSD) - bestimmen. Pixelarraydetektoren und PSD können aber auch Intensitäten messen.

Mit Pixelarraydetektoren und positionssensitiven Detektoren können Statistik und Mittelungsverfahren ausgeführt werden, indem die Detektoren mehrfach ausgelesen werden. Diese Verfahren erhöhen die Messgenauigkeit.

Die Sensorsignale (+-)(ΔN(TE+) - ΔN(TM+)) und (+-)(ΔN(TE-) - ΔN(TM-)) stellen keine temperaturkompensierten Sensorsignale dar und zeigen infolge der Differenzbildung zudem geringere Sensitivität als die referenzierten mit hoher Sensitivität ausgestatteten Sensorsignale ΔN(TE) und ΔN(TM) (oder zum Beispiel (Aenderungen der) Auskopplungswinkel)

Die Messung von vier Moden erlaubt ebenfalls eine bessere Kompensation von Temperaturdrift bzw. Porendrift. Die verschiedenen zur Anwendung kommenden Algorithmen sind in der PCT-Anmeldung PCT/CH98/00389 beschrieben. Als Messignal kann zum Beispiel die Aenderung der Schichtdicke des wellenleitenden Films (im Dreischichtwellenleitermodell) oder die Aenderung der (Gesamt)Schichtdicke der Adlayer (chemofunktionale Schicht plus Bindungsschicht) dienen. Es kann mit einer oder mehreren Wellenlängen gearbeitet werden.

Auch mit den vorliegenden Wellenleitergitterstrukturen können - wie schon in der PCT-Anmeldung PCT/CH98/00389 beschrieben - Fluoreszenz-, Lumineszenz-, Chemitumineszenz- und Phosphoreszenzmessungen durchgeführt werden. Diese stellen ebenfalls weitgehend temperaturunabhängige Messungen dar. Es kann z.B. für das 'direct sensing' die gleiche Wellenlänge wie für die Fluoreszenz- oder Lumineszenzanregung genommen werden. Es können jedoch für das 'direct sensing' und für die Fluoreszenz- oder Lumineszenzanregung auch verschiedene Wellenlängen verwendet werden. Bei der Markierungsmessung (z.B. Fluoreszenz, Lumineszenz etc.) befindet sich vor dem Detektor ein Wellenlängenfilter, das nur das Markierungslicht (z.B Fluoreszenzlicht, Lumineszenzlicht etc) durchlässt.

Das Fluoreszenz- oder Lumineszenzsignal kann mit einem oder mehreren Detektoren oder mit einem Pixelarraydetektor (gegebenenfalls mit Abbildungsoptik oder einem Array von Abbildungsoptiken) detektiert werden. Beispielsweise kann unter der Wellenleitergitterstruktur der Messkopf zum Markierungsnachweis und der Messkopf für den markierungsfreien Nachweis ausgetauscht werden, es kann jedoch beispielsweise auch nur das Linsensystem geändert oder ausgetauscht und das Wellenlängenfilter, das für den Markierungsnachweis notwendig ist, entfernt werden.

Ein anderes Messverfahren beruht auf einer **Streulichtmessung** im Wellenlängen-Scanmodus (siehe auch weiter unten) oder im Einfallswinkel-Scanmodus (siehe auch weiter unten), wobei das Streulicht der Wellenleitergitterstruktureinheit(en) über eine Linse oder ein Linsensystem auf einen eindimensionalen oder zweidimensionalen Array von Detektoren (z.B. auf einen Pixelarraydetektor) abgebildet und während eines Scanvorgangs gemessen wird. Die Scanvorgänge können wiederholt werden.

Beim Wellenlängen-Scanmodus wird die Wellenlänge der einfallenden (aufgeweiteten) Lichtfelder (des einfallenden aufgeweiteten Lichtstrahls) bei festem Einfallswinkel durchgestimmt und Streulichtbilder der Wellenleitergitterstruktur in Funktion der Wellenlänge aufgenommen. Dabei kann sich z.B. die Wellenlänge mit zeitlich konstanter Rate ändern, während parallel dazu (synchron) mit konstanter Rate die Streulichtbilder aufgenommen (und ausgelesen) werden. Beim Wellenlängenscan erfolgt bei einer bestimmten Wellenlänge Gittereinkopplung für das jeweilige Gitter(die Einkopplungsbedingung ist erfüllt) und das Streulicht nimmt markant zu. Die Streulichtintensität durchläuft beim Wellenlängenscan während der Gittereinkopplung eine Resonanz. Bestimmt wird mit oder ohne Verwendung von Schwerpunktsalgorithmen jene Wellenlänge (=Resonanzwellenlänge), die dem (gewichteten) Resonanzmaximum entspricht. Aus der Verschiebung der Wellenlänge für Modenanregung kann auf die Bindung (Massenbelegung) geschlossen werden. Die Resonanzwellenlängen für die verschiedenen Einkopplungsgitter sind zur Verminderung von Crosstalk im allgemeinen verschieden (können jedoch auch zusammenfallen).

Da die Sensitivität für die Massenbelegung (Anlagerung einer Zusatzschicht) von der Wellenlänge abhängt, kann diese Abhängigkeit gegebenenfalls bei der Auswertung mathematisch berücksichtigt werden.

Als Lichtquelle (Strahlungsquelle) wird ein abstimmbarer Laser (oder eine abstimmbare Laserdiode oder ein abstimmbarer VCSEL) verwendet. Weitere abstimmbare Lichtquellen sind im Abschnitt "Wellenlängen-Scanmodus" (siehe weiter unten) erwähnt. Vorzugsweise wird die Wellenlänge aller einfallenden Lichtfelder simultan gleich geändert.

Die Figuren 3a,3b,4a,4b,5a,5b sind jetzt auch so zu verstehen, dass die Linse das Streulicht (oder das Fluoreszenz(Lumineszenz)licht) auf den Pixelarraydetektor abbildet. Bei Fluoreszenzmessungen braucht es allerdings vor dem Detektor noch ein Wellenlängenfilter, um das Streulicht abzublocken.

Die Auskopplungsgitter der Wellenleitergitterstruktureinheit(en) sollen bei diesem Messmodus die ausgekoppelten Lichtstrahlen vorzugsweise nicht auf den Pixelarraydetektor lenken. Das Auskopplungsgitter ist z.B. identisch mit dem Einkopplungsgitter und schliesst z. B. direkt an das Einkopplungsgitter an, woraus sich dann praktisch aus Einkopplungsgitter und Auskopplungsgitter ein einziges Gitter ergibt. Der Wellenleiter kann (muss nicht) lichtabsorbierend sein, kann aber auch nur zwischen den Gittern lichtabsorbierende Bereiche aufweisen.

Zwei Wellenleitergitterstruktureinheiten können auch bei diesem Messmodus gegeneinander referenziert werden.

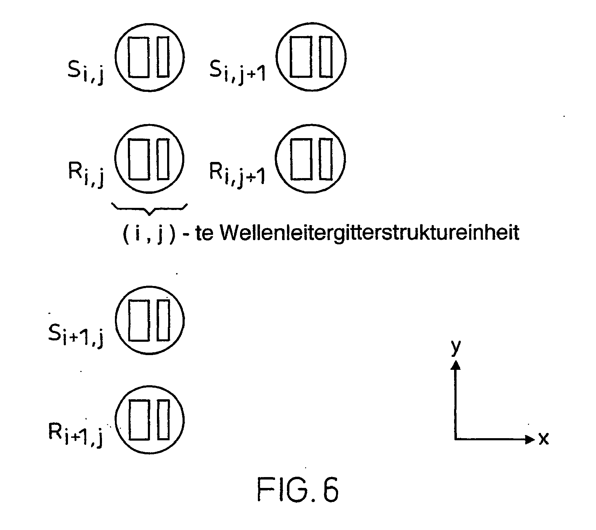

Figur 6 zeigt einen Array von Wellenleitergitterstruktureinheiten (i,j) bestehend aus einem Signalpfad S(i,j) und einem Referenzpfad R(i,j). Ein Pfad enthält ein Einkopplungsgitter (als grosses Rechteck eingezeichnet) und ein Auskopplungsgitter (als kleines Rechteck eingezeichnet) mit Gitterstrichen parallel zur y-Achse und eine chemofunktionale Schicht (als Kreisfläche eingezeichgnet), die gegebenenfalls Einkopplungsgitter und Auskopplungsgitter vollständig belegt (oder nur das Einkopplungsgitter oder das Auskopplungsgitter oder die Region zwischen Einkopplungsgitter und Auskopplungsgitter oder den gesamten Pfad belegt). Auf dem Signalpfad (Sensorpfad) befindet sich die chemofunktionale Signalschicht und auf dem Referenzpfad die chemofunktionale Referenzschicht. Der Referenzpfad R(i,j) kann (muss nicht) bei jeder Wellenleitergitterstruktureinheit (i,j) vorhanden sein. Der Wellenleiter (die Wellenleiterstruktur) kann (muss nicht) lichtabsorbierend sein oder kann zwischen zwei Wellenleitergittern oder zwischen zwei Wellenleitergitterstruktureinheiten (zumindest teilweise) lichtabsorbierend sein. Die Lichtabsorption hilft, Crosstalk zu vermindern.

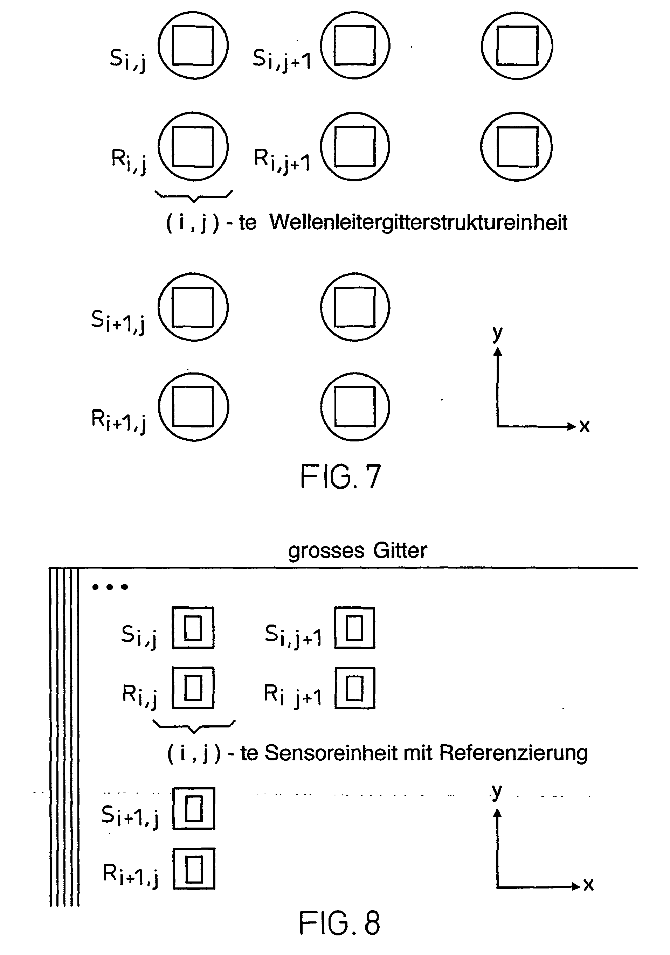

Figur 7 zeigt einen Array von Wellenleitergitterstruktureinheiten (i,j) bestehend aus einem Signalpfad S(i,j) und einem Referenzpfad R(i,j). Ein Pfad enthält ein Wellenleitergitter (als Rechteck eingezeichnet) mit Gitterstrichen parallel zur y-Achse und eine chemofunktionale Schicht (als Kreisfläche eingezeichgnet), die das Wellenleitergitter vorzugsweise vollständig (oder auch nur teilweise) belegt (die den Pfad ausserhalb des Wellenleitergitters oder allgemein zumindest teilweise den Pfad belegt). Auf dem Signalpfad (Sensorpfad) befindet sich die chemofunktionale Signalschicht und auf dem Referenzpfad die chemofunktionale Referenzschicht. Der Referenzpfad R(i,j) kann (muss nicht) bei jeder Wellenleitergitterstruktureinheit (i,j) vorhanden sein. Die Wellenleiterstruktur kann absorbierend sein oder kann zumindest zwischen zwei benachbarten Wellenleitergitterstruktureinheiten absorbierend sein. Die Lichtabsorption hilft, Crosstalk zu vermindern.

Streulichtmessungen sind auch im Einfallswinkel-Scanmodus möglich. Der Messaufbau für den Einfallswinkel-Scanmodus ist weiter unten beschrieben. Bei Streulichtmessungen wird vorzugsweise jedoch - durch geeignete Wahl der Gitterperioden - darauf geachtet, dass kein gebeugtes Licht auf den Pixelarraydetektor fällt.

Beim Einfallswinkel-Scanmodus wird anstelle der Durchstimmung der Wellenlänge bei fester Wellenlänge (oder gegebenenfalls bei einer festen Wellenlängenverteilung) der Einfallswinkel des einfallenden (aufgeweiteten) Lichtstrahls winkelmässig durchgestimmt. Dabei kann sich der einfallende (aufgeweitete) Lichtstrahl winkelmässig bezüglich der festen Wellenleitergitterstruktur ändern oder die Wellenleitergitterstruktur (samt Linsen- und Detektionssystem) winkelmässig bezüglich des festen einfallenden (aufgeweiteten) Lichtstrahls ändern.

Aus einer Wellenleitergitterstruktureinheit lässt sich auch nur ein Einkopplungsgitter (und gegebenenfalls ein Auskopplungsgitter) generieren, indem alle anderen Gitter mit Modulationsstärke = 0 versehen werden. Ein Array von chemofunktionalen Schichten kann auf dem Gitter, neben dem Gitter oder zwischen den Gittern angebracht werden. Es wird ein Lichband (ein aufgeweiteter Lichtstrahl) über das Einkopplungsgitter eingekoppelt und das Streulicht und/oder Fluoreszenz(Lumineszenz)licht am Array mit einem Array von Detektoren oder mit einem Pixelarraydetektor (z.B. CCD) mit Abbildungsoptik gemessen. Das Streulicht und/oder Fluoreszenz(Lumineszenz)licht kann aber auch über einen Array von Glasfasern (Glasfaserbündeln) (mit oder ohne aufgesetzten Linsen) mit guter Lichtausbeute aufgefangen und einem Array von Detektoren (z.B Pixelarraydetektor) zugeführt werden. Vorteilhafterweise sind die Faserbündel in einer Reihe angeordnet. Zeitaufgelöste Detektion erlaubt die Messung von Bindungsvorgängen.

Im Fall von Streulicht- und Fluoreszenzmessungen kann der Messkopf (Linse und Detektor) beide Messmodi ausführen. Bei der Fluoreszenz(Lumineszenz)messung braucht es jedoch noch ein Wellenlängenfilter zwischen der(den) Wellenleitergitterstruktur(en) und dem Detektor.

Mit der Fluoreszenztechnik als auch mit der Streulichttechnik können Sandwichassays, Competionassays und weitere Mehrstufenassays durchgeführt werden. Im Fall von der Streulichttechnik kommen als Markierungssubstanzen Index-Label (z.B. Goldkügelchen, Metallkügelchen, Kunststoffkügelchen, Latexkügelchen, biochemische und biologische Substanzen und Fragmente) zum Einsatz. Es können neben Direktmessungen auch Sandwichassays, Competitionassays und mehrstufige Assays im markierungsfreien Betrieb als auch im Markierungsbetrieb durchgeführt werden. Bei Fluoreszenzmessungen (Lumineszenzmessungen) kommt ein Fluoreszenz(Lumineszenz)marker zum Einsatz.

Eine Fluoreszenz(Lumineszenz)anregung via evaneszente Welle stellt einen dissipativen Effekt dar und kann deshalb als Aenderung der komplexen effektiven Brechzahl aufgefasst werden (Imaginärteil der komplexen effektiven Brechzahl der Anregungswelle ändert sich). Die Fluoreszenzmessung stellt sozusagen eine indirekte Messung der (Aenderung der) komplexen effektiven Brechzahl der Anregungswelle (bzw. (der Aenderung) des Imaginärteils der komplexen effektiven Brechzahl der Anregungswelle) dar.

Der markierungsfreie Nachweis kann im (i) Wellenlängen-Scanmodus, (ii) Einfallswinkel-Scanmodus und (iii) Auskopplungswinkel-Scanmodus erfolgen. Die Scanmodi sind nachfolgend beschrieben. Messtechniken, die auf Scanmodi beruhen, zeichnen sich durch einen hohen dynamischen Bereich aus.

In (i) und (ii) erfolgt das Durchfahren (Durchstimmen) des Scanintervalls, das die Resonanzkurve (Vertiefung und/oder Ueberhöhung der gemessenen Lichtintensität in Funktion des Scanparameters) enthält, vorzugsweise kontinuierlich (d.h. also nicht in einem 'Stop and Go'-Betrieb). Der Scanvorgang ist mit der durch den Detektor vorgenommenen Messung der Intensität(en) des Lichtfelds (der Lichtfelder) zeitlich oder über Triggerpulse synchronisiert. Gerade hier ist es wichtig, dass der Scanvorgang mit nicht mechanisch bewegten Komponenten zum Beispiel durch Einsatz einer abstimmbaren Laserdiode oder eines abstimmbaren VCSEL oder eines über die Temperatur abstimmbaren Festkörperlasers beim Wellenlängen-Scanmodus oder durch Einsatz eines elektrooptischen oder akustooptischen Lichtsirahtdeflektors beim Einfallswinkel-Scanmodus abläuft, da im Gegensatz zu einem mechanischen Scanvorgang keine bewegten Massen involviert sind. Bewegte Massen verursachen Beschleunigungsprobleme und verlangsamen den Scanvorgang, was die Ausnutzung von Mittelungsverfahren reduziert, da pro Zeiteinheit weniger Scans durchgeführt werden können. Beim Scanvorgang initiert meistens ein Starttriggerimpuls den Beginn der Aufnahme der Lichtfelder. Triggerimpulse, die mit dem Scanparamter (digital oder analog) korrelieren, können während eines Scandurchgangs weitere Lichtfeldaufnahmen des Detektors auslösen. Es ist aber auch eine rein zeitliche Synchronisierung möglich.

Bei den Scanmodi kommen für die Detektion des Lichts eindimensionale oder zweidimensionale Arrays von Detektoren oder Arrays von positionssensitiven Detektoren, vorzugsweise eindimensionale oder zweidimensionale Pixelarraydetektoren (CCD, Photodiodenarray, CMOS-Array, etc.) zum Einsatz.

Vorteilhaftweise - aber nicht unbedingt notwendig - wird abgestrahltes Licht beobachtet, das nicht in Richtung des reflektierten Lichtstrahls abstrahlt. Dadurch lässt sich der Background Noise, der durch die Reflexion des einfallenden Lichtstrahls an der Substratunterseite der Wellenleitergitterstruktur und an der Flüssigkeitsoberfläche der Probe entsteht, reduzieren. Auch brauchen keine Antireflexschichten auf die Unterseite der Wellenleitergitterstruktur aufgebracht werden. Auch können auch Keilsubstrate vermieden werden.

Die Messung der abgestrahlten nichtreflektierten Lichtwelle (UV, VIS, IR) erfolgt beim Einkopplungsgitter über eine Beugungsordnung, die nicht der Richtung des reflektierten Lichtstrahls entspricht, oder gar über ein zweites Gitter. Das zweite Gitter kann (muss aber nicht) eine andere Gitterperiode aufweisen als das erste Gitter. Die Lichtauskopplung über das zweite Gitter kann ebenfalls über verschiedene Beugungsordnungen erfolgen.

Die Messung der ersten Beugungsordnung in Reflexion hat auch den Vorteil, dass mit kleineren Gitterlinienzahlen gearbeitet werden kann. Es kann auch erreicht werden, dass der Auskopplungswirkungsgrad über die zweite Beugungsordnung (z.B. beim Einkopplungsgitter) klein ist. Ein Sinus(Kosinus)gitter hat zum Beispiel nur eine plus/minus erste Beugungsordnung. Es kann auch nur mit einem grossen Gitter gearbeitet werden. Während eines Scanvorgangs reduziert sich bei Lichteinkopplung die Intensität der direkt gebeugten Beugungsordnungen (inklusive nullter Beugungsornung) in Reflexion und Transmission. Bei der direkt gebeugten Beugungsordnung wird nicht die zuerst über eine Beugungsordnung eingekoppelte und dann über eine Beugungsordnung ausgekoppelte Lichtwelle beobachtet. Insbesondere reduziert sich bei Lichteinkopplung die Intensität der direkten (plus/minus) ersten Beugungsordnung in Reflexion. Diese Intensitätsverminderung der direkten (plus/minus) ersten Beugungsordnung in Reflexion hat Resonanzcharakter und kann beim Wellenlängen-Scanmodus und beim Einfallswinkel-Scanmodus gemessen werden. Die über die (minus) zweite Beugungsordnung ausgekoppelte Lichtwelle ist gegenüber der direkten (plus/minus) ersten Beugungsordnung lateral leicht versetzt (typischerweise um eine Kopplungslänge). Zudem kann durch geeignete Wahl der Gitterstruktur erreicht werden, dass der Kopplungswirkungsgrad der zweiten Beugungsordnung klein ist (Ein Sinusgitter zeigt keine (plus/minus) zweite Beugungsordnung). Es können nicht absorbierende als auch absorbierende Wellenleiter zum Einsatz kommen.

Die Messung der ersten (zweiten) Beugungsordnung (in Reflexion) kann sich auf ein (grosses) unidiffraktives Gitter oder aber auch auf ein Spatialfrequenzgitter eines (grossen) multidiffraktiven Gitters beziehen.

Die Modulation von zumindest einem Gitter einer Wellenleitergitterstruktureinheit ist vorzugsweise derart gewählt, dass das bei einer bestimmten Wellenleitergitterstruktureinheit (bzw. Sensorstelle) eingekoppelte Licht nicht bis zur benachbarten Wellenleitergitterstruktureinheit (bzw. Sensorstelle) propagiert. Es soll also Crosstalk vermieden werden, denn sonst würde beispielsweise die benachbarte Wellenleitergitterstruktureinheit eine ausgekoppelte oder abgestrahlte Lichtwelle aufweisen, obschon es bei dieser benachbarten Wellenleitergitterstruktureinheit zu keiner Lichteinkopplung kommt.

Eine stärkere Gittermodulation bewirkt, dass das eingekoppelte Licht über eine kurze Wegstrecke bereits vollständig ausgekoppelt wird. Es kann zumindest ein Gitter der Wellenleitergitterstruktureinheit stärker moduliert sein. Gegebenenfalls kann sich zwischen den Wellenleitergitterstruktureinheiten jeweils ein Stoppgitter (mit stärkerer Modulation) oder ein Stopptaper oder eine Stopprille befinden. Die Wellenleitergitterstruktureinheit kann aus einem einzelnen Gitter, ein oder mehreren Einkopplungsgittern und ein oder mehreren Auskopplungsgittern bestehen. Die Gitter können unidiffraktiv oder multidiffraktiv sein. Die Wellenleitergitterstruktureinheiten können auch zusammenhängen und somit z.B. ein grosses Gitter bilden. Die chemoempfindlichen (chemofunktionalen) Schichten können dann matrixförmig auf einem grossen Wellenleitergitter angeordnet sein (ohne sich gegenseitig zu berühren).

Figur 8 zeigt einen Array von Sensoreinheiten (i,j) bestehend aus einem Signalpfad S(i,j) und einem Referenzpfad R(i,j) auf einem grossen (unidiffraktiven, bidiffraktiven oder multidiffraktiven) Wellenleitergitter mit Gitterstrichen parallel zur y-Achse. Beim multidiffraktiven Gitter sind zwei, drei, vier oder mehr verschiedene Spatialfrequenzgitter (mit gleicher oder unterschiedlicher Orientierung) überlagert. Ein Pfad enthält eine (bio)chemofunktionale Schicht (als kleines Recheck eingezeichnet), die gegebenenfalls eine Abstandschicht (Zwischenschicht)(als grosses Rechteck eingezeichnet) belegt. Der Referenzpfad R(i,j) muss nicht unbedingt bei jeder Sensoreinheit (i,j) vorhanden sein. Die (bio)chemofunktionale Signalschicht befindet sich auf dem Signalpfad und die (bio)chemofunktionale Referenzschicht auf dem Referenzpfad. Der Wellenleiter kann lichtabsorbierend oder zwischen zwei Sensorstellen lichtabsorbierend oder nicht lichtabsorbierend sein. Die chemofunktionalen Signalschichten und Zwischenschichten können auch kreisförmig und/oder gleich gross sein.

Die in den Figuren 2 bis 8 gezeigten (chemofunktionalen) Wellenleitergitterstrukturen können in allen Scanmodi eingesetzt werden. Das Aufbringen der chemofunktionalen Schichten erfolgt z.B. mit einem Spotter oder einem 'contact-printing'-Roboter oder einem 'ink-jet'-Roboter oder einem 'liquid handler. Die Sensor Chips können in der Genomik und Proteomik eingesetzt werden.

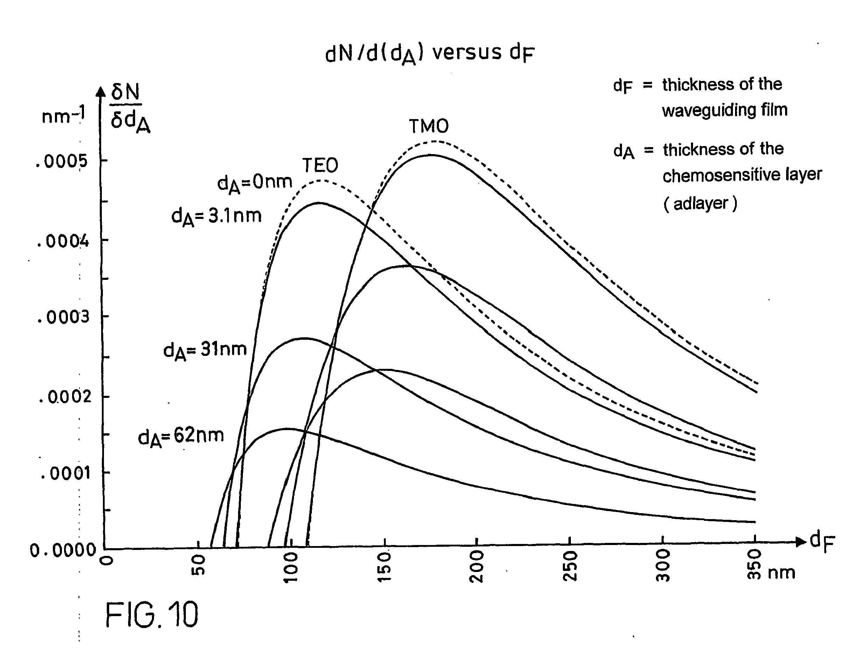

Bei der matrixförmigen Belegung eines grossen Wellenleitergitters mit chemofunktionalen Signalschichten (mit oder ohne chemofunktionalen Referenzschichten) weisen vorzugsweise Gitterbereiche mit chemoempfindlichen Schichten andere effektive Brechzahlen auf als die effektive Brechzahlen in Gitterbereichen ausserhalb der chemoempfindlichen Schichten. Dies soll auch gelten, wenn in den Gitterbereichen ausserhalb der chemoempfindlichen Schichten eine Passivierungsschicht vorhanden ist, um nicht spezifische Bindung (NSB) zu unterdrücken. Damit soll gewährleistet sein, dass die Lichteinkopplung in der Region der chemoempfindlichen Schichten unter einem anderen Resonanzscanparameter (Resonanzeinfallswinkel oder Resonanzwellenlänge) erfolgt als in der Region ausserhalb der chemoempfindlichen Schicht, wobei die Resonanzscanparamter mehr als eine Resonanzhalbwertsbreite, typischerweise mehrere Resonanzhalbwertbreiten, auf der Scanparameterachse voneinander entfernt sein sollen. Dies vereinfacht die (laterale) Identifikation der chemoempfindlichen Schichten mit dem Detektionssystem. Die Dicke der chemoempfindlichen Schichten kann zum Beispiel grösser (oder kleiner) als jene der Passivierungsschicht sein. Die chemoempfindliche Schicht kann auch auf einer Zwischenschicht sitzen. Oder umgekehrt, es kann auch die Passivierungsschicht auf einer Zwischenschicht sitzen. Ist die Dicke der Passivierungsschicht (plus eventuell vorhandener Zwischenschicht) grösser als die Dicke der chemoempfindlichen Schicht, kann die hohe Sensitivität für selektive Bindungen an der Oberfläche der chemoempfindlichen Schicht gewährleistet werden (siehe Figur 10). Unter den chemoempfindlichen Schichten kann sich auch eine Linker-Schicht (oder Linker-Schichten) befinden.

Die Passivierungsschicht kann auch lichtabsorbierend sein. Beispielsweise kann die Passivierungsschicht mit Farbstoffen versehen sein.

Die Passivierungsschicht hilft nicht spezifische Bindung (NSB) zu vermeiden oder (wesentlich) zu vermindern. Sind die Resonanzscanparameter von chemofunktionaler Schicht und Passivierungsschicht einmal voneinander getrennt, bleiben sie voneinander getrennt, da die Passivierungschicht NSB unterdrückt. Während eines Scanvorgangs werden somit die beiden Resonanzscanparameter zu unterschiedlichen Zeiten auf der Scanparameterachse durchquert, was hilft, Crosstalk zu unterdrücken.

Ohne Passivierungsschicht tritt NSB auf und es kann im Verlauf der Messung zu einer Koinzidenz der beiden Resonanzscanparamter (Resonanzscanparameter im (Wellenleitergitter)Bereich mit chemofunktionaler Signalschicht und Resonanzscanparameter im (Wellenleitergitter)Bereich ohne chemofunktionale Signalschicht) auf der Scanparameterachse kommen, auch wenn anfänglich die effektive Brechzahl im Bereich der chemfunktionalen Signalschicht grösser als jene im Bereich ausserhalb der chemofunktionalen Signalschicht ist. Die Ueberlegungen gelten auch, wenn anstelle der chemofunktionalen Signalschicht die chemofunktionale Referenzschicht betrachtet wird. Fallen die beiden Resonanzscanparameter bei einem Scanvorgang zeitlich zusammen, koppelt (im Beispiel von einem grossen Wellenleitergitter) Licht von einem Bereich ohne chemofunktionaler Signalschicht in einen (anschliessenden) Bereich mit chemofunktionaler Signalschicht und von einem Bereich mit chemofunktionaler Signalschicht in einen (anschliessenden) Bereich ohne chemofunktionale Signalschicht mehr oder weniger zeitgleich hinüber, was zu einer Störung der vom Detektor ortsaufgelöst aufgenommenen Resonanzkurven (ortsabhängige Resonanzkurve(n), die dem Bereich mit chemofunktionaler Signalschicht zugeordent ist (sind)), und ortsabhängige Resonanzkurve(n), die dem Bereich ohne chemofunktionale Signalschicht zugeordnet (ist)sind) führt und damit zu Crosstalk führt. Infolge des Hinüberkoppelns des Lichts sind die Resonanzkurven (z.B. Maximum oder Minimum), die z.B. dem Bereich mit chemofunktionaler Signalschicht entsprechen, nicht mehr richtig ausgeprägt. Es kann zu einer Verformung der Resonanzkurve kommen.

Das Hinüberkoppeln auf einem grossen Wellenleitergitter , d.h. Lichteinkopplung und anschliessende Lichtauskopplung, ist typischerweise um eine Kopplungslänge zueinander versetzt, da der Mode bei der Einkopplung zuerst 'aufgebaut' werden muss. Messtechnisch interessante Bereiche sind - in Modenausbreitungsrichtung - der Uebergang von einem Bereich ohne chemofunktionaler Signalschicht in den Bereich mit chemofunktionaler Signalschicht bzw. die erste oder ersten paar Kopplungslängen in Modenausbreitungsrichtung nach dem Uebergang (=Region I) und - in Modenausbreitungsrichtung der Uebergang von einem Bereich mit chemofunktionaler Signalschicht in den Bereich ohne chemofunktionale Signalschicht bzw. die erste oder ersten paar Kopplungslängen in Modenausbreitungsrichtung nach dem Uebergang (=Region III). Zwischen beiden Uebergängen wird die Region II definiert. Uebergänge in Richtung Modenausbreitung (d.h. die Ausformung des Randes der chemofunktionalen Signalschicht) sollen möglichst stufenförmig sein, vorzugsweise aber bedeutend kleiner als die Kopplungslänge sein. Zum Beispiel lassen sich mit einem Spotter chemofunktionale Signalschichten mit recht scharf definierten Uebergängen herstellen. Dies ist wichtig bei Messungen der Regionen I und III. Der Abstand zwischen zwei chemofuntionalen Signalschichten in Modenausbreitungsrichtung ist typischerweise auch ein oder mehrere Kopplungslängen. In der Region I tritt bei Modenanregung im Bereich der chemofunktionalen Signalschicht Lichtabschwächung in der direkt gebeugten Beugungsordnung auf, solange kein Hinüberkoppeln stattfindet. In der Region III tritt bei Modenanregung im Bereich der chemofunktionalen Signalschicht (ohne Berücksichtigung von Interferenz) Lichterhöhung (allgemein: Lichtänderung)(in Richtung der direkt gebeugten Beugungsordnung) auf, da Hinüberkoppeln stattfindet. In der Region II tritt bei Modenanregung Hinüberkoppeln auf und der Grad der Lichtänderung (in Richtung der direkt gebeugten Beugungsordnung) hängt vom Lichtabsorptionskoeffizienten des Wellenleiters, von den Beugungswirkungsgraden (bzw. von den Gitterformen) und von Interferenzparametern ab. Es können die Lichtintensitäten von Region I oder II oder III oder von zwei Regionen oder von allen drei Regionen gemessen werden. Es kann auch die Lateralverschiebung des Lichtschwerpunkts bei Resonanz gemessen werden. Dies kann insbesondere interessant sein, wenn die Ausdehnung der chemofunktionalen Signalschicht in Modenausbreitungsrichtung nur ein oder ein paar Kopplungslängen beträgt.

Bleiben der Resonanzscanparamter für den Wellenleitergitterbereich mit chemofunktionaler Signalschicht und der Resonanzscanparameter für den Wellenleitergitterbereich ohne chemofunktionaler Signalschicht mindestens eine oder mehrere Resonanzhalbwertsbreiten auf der Scanparameterachse voneinander entfernt, kann dieser Beitrag zum Crosstalk reduziert (vermieden) werden. Es tritt dann in der Region I Lichtverminderung und in der Region III Lichterhöhung (ohne Berücksichtigung von Interferenz) auf. Für die Detektion der Lichtverminderung reicht in Modenausbreitungsrichtung eine Ausdehnung der chemofunktionalen Signalschicht von ein oder ein paar Kopplungslängen.

Erfolgt das Hinüberkoppeln des Lichts in den Bereich mit chemofunktionaler Signalschicht (=Region I) nicht zu einem Zeitpunkt, in dem gerade die Resonanzkurve des Bereichs mit chemofunktionaler Schicht gemessen wird, ist die Störung nicht sehr gross, da im Prinzip im Bereich der chemofunktionalen Signalschicht ein ausserhalb der Resonanzkurve liegender Sidepeak auftritt und dieser bei der Bestimmung des Resonanzscanparameters (der Resonanzposition) ausgeblendet werden kann.

Ist keine Passivierungschicht vorhanden (es kann also NSB auftreten), sollte die Dicke der chemofunktionalen Schicht (bzw. die Gesamtdicke von chemofunktionaler Schicht und Zwischenschicht) so gewählt werden, dass die Resonanzscanparameter der beiden Bereiche (Bereich mit und ohne chemofunktionaler Schicht) immer ein oder mehrere Resonanz(halbwerts)breiten auf der Scanparameterachse voneinander getrennt sind. Die Resonanzbreite ist umgekeht proportional zur Kopplungslänge. Eine starke Gittermodulation bzw. Lichtabsorption im Wellenleiter verkürzen die Kopplungslänge und grössern die Resonanz(halbwerts)breite.

Obige Ueberlegungen werden im speziellen wichtig, wenn der Bereich mit chemofunktionaler Signalschicht in Modenausbreitungsrichtung eine Ausdehnung von ein oder ein paar Kopplungslängen hat bzw. die Messung (auch bei grösseren Ausdehnungen) in Modenausbreitungsrichtung im Bereich der ersten oder ersten paar Kopplungslängen nach der Berandung der chemofunktionalen Signalschicht vorgenommen wird. Der Abstand von zwei chemofuntionalen Signalschichten in Modenausbreitungsrichtung ist typischerweise auch ein oder mehrere (typischerweise 1 bis 10 oder 1 bis 100) Kopplungslängen. Chemofunktionale Signalschichten mit Ausdehnung von nur einer oder ein paar Kopplungslänge(n) führen zu höher verdichteten Arrays.

Bei grösserer Ausdehnung der chemofunktionalen Schicht auf einem grossen Wellenleitergitter überlagern sich nach ungefähr einer Kopplungslänge in Modenausbreitungsrichtung die direkt gebeugte Beugungsordnung mit der ausgekoppelten Beugungsordnung. Der Beugungswirkungsgrad für die verschiedenen Beugungsordnungen und Interferenzparameter bestimmen, wie stark die Resonanz ausgeprägt ist. Beispielsweise kann darauf geachtet werden, dass die zweite ausgekoppelte Beugungsordnung sehr schwach ist. Eine grössere Ausdehnung der chemofunktionalen Schicht auf dem Wellenleitergitter entspricht bei gleicher Abbildungsvergrösserung einer grösseren Fläche auf dem Pixelarraydetektor. Es kann deshalb z.B. die Lichtintensität von einer grösseren Anzahl Pixels gemittelt werden oder von einer grösseren Anzahl Pixels jeweils eine Resonanzkurve aufgenommen werden. Im letzteren Fall können entweder zuerst die Resonanzkurven gemittelt und dann der Resonanzscanparameter ermittelt werden oder es können zuerst aus jeder Resonanzkurve der Resonanzscanparameter ermittelt und anschliessend die Resonanzscanparameter gemittelt werden.

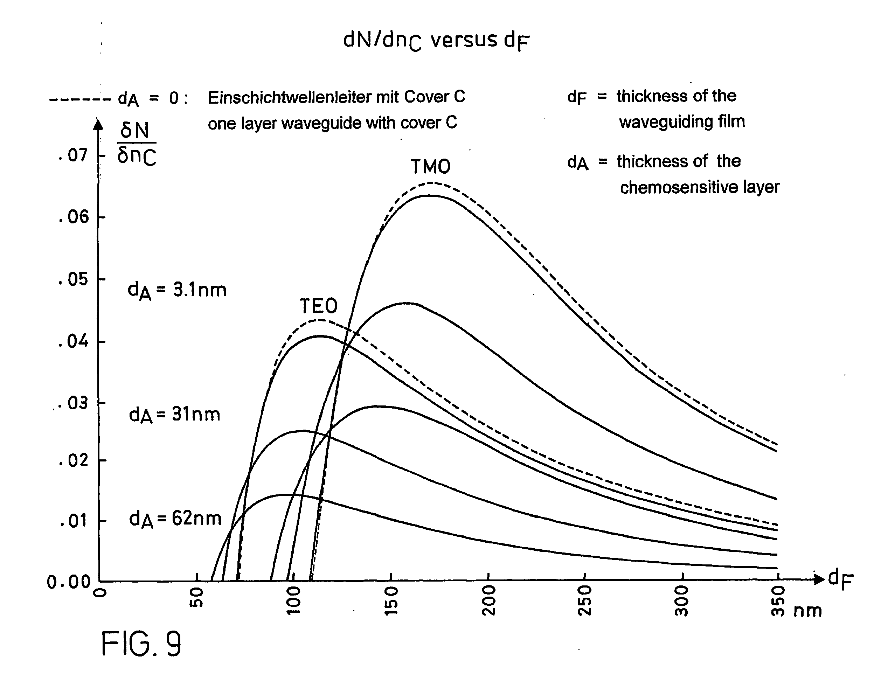

Die Passivierungsschicht kann auch als chemofunktionale Referenzschicht aufgefasst werden, die die chemofunktionale Sensorschicht umschliesst. Bei unterschiedlicher Dicke der Passivierungsschicht und der chemofunktionalen Signalschicht können (sollten) die unterschiedlichen Sensivitäten (siehe Figuren 9 und 10) berücksichtigt werden.

Es besteht auch die Möglichkeit zuerst den Sensor Chip flächendeckend mit einer Passivierungsschicht zu belegen und dann lokal (eventuell im Array-Format) die chemoempfindlichen Schichten (mit Linkerlayer)(gegebenenfalls mit einem Spotter) aufzubringen. Die Kombination Passivierungsschicht mit chemoempfindlicher Schicht ist immer dicker als die Passivierungsschicht alleine.

Es besteht auch die Möglichkeit, zuerst die chemoempfindlichen Schichten aufzubringen und anschliessend die freien Bereiche (Bereiche, die nicht mit einer chemoempfindlichen Schicht versehen sind) zu passivieren. Der Sensor Chip kann auch zuerst mit einer Linkerlayer versehen werden. Das Passivierungsmaterial kann auf den gesamten Sensor Chip aufgebracht werden, haftet jedoch nur an die freien Bereiche: Insbesondere verdeckt die Passivierungsschicht nicht die Bindungsstellen der chemoempfindlichen Schicht oder kann aber zumindest weggewaschen werden.

Zur Identifikation der chemoempfindlichen Schichten können auch am Sensor Chip angebrachte Markierungen oder Fenster dienen, die z.B. auf einen Pixelarraydetektor mit Linsenoptik abgebildet werden.

Es kann sich jedoch auch auf der Unterseite des Sensor Chips oder am wellenleitenden Film (am Interface Substrat/wellenleitender Film bzw. am Interface Cover/wellenleitender Film) oder in der Wellenleiterstruktur eine nichttransparente (bzw. lichtabsorbierende) Schicht (z.B. aus Chrom) mit Fenstern befinden. Die Fenster liegen den chemoempfindlichen Schichten gegenüber. Es kann (muss aber nicht) eine chemoempfindliche Schicht zumindest ein ganzes Fenster bedecken. Das einfallende Licht kann durch die Fenster auf die Wellenleitergitterstruktur fallen. Es kann auch sein, dass das Substrat oder eine Schicht oder eine Schicht der Wellenleiterstruktur lichtabsorbierend ist und an bestimmten Stellen transparente Fenster aufweist (und eventuell auch die Gitterstruktur trägt). Es gibt zum Beispiel photoempfindliche (UVempfindliche, IR-empfindliche, VIS-empfindliche) Substrate und Schichten (aus Glas, Polymermaterialien (z.B. Kunststoffe), Sol-Gel-Materialien, Ormocere), deren Transparenz strukturierbar ist.

Die nichttransparente Schicht mit Fenstern an der Unterseite des Sensor Chips (Wellenleitergitterstruktur) kann auch als Folie (aufgeklebt oder nur durch Adhäsionskräfte haltend) vorliegen oder durch Siebdruck oder durch Bedampfung mit einer Blendenvorrichtung, die die Fensterpositionen definieren, oder durch Photolithographie hergestellt werden. In diesem Fall soll das Substrat eher dünn sein (z.B. 0.1 mm bis 1 mm).

Das ausserhalb der Fenster abgestrahlte Licht wird von der nichttransparenten Schicht (vom Substrat) absorbiert und/oder die geführte Lichtwelle wird durch diese Schicht oder durch das (teilweise) lichtabsorbierende Substrat gedämpft. Die Ausdehnung eines Fensters in Modenausbreitungsrichtung ist typischerweise ein oder meherere Kopplungslängen (kann aber auch kleiner-als eine Kopplungslänge sein). Die Wellenleitergitterstruktur kann (muss nicht) ein grosses Wellenleitergitter sein.

Prinzipiell kann auch die Ebene der nichttransparenten Schicht mit den Fenstern auf die Detektionsebene abgebildet werden.

Das Substrat kann ähnlich zu einem extrem groben Gitter (kein Beugungsgitter) auch reliefmässig gerastert sein. Dieses extrem grobe Gitter kann z.B. ein Rechteckgitter sein. Die eigentliche Wellenleitergitterstruktur befindet sich zumindest teilweise in den Mulden (unteres Niveau) oder zumindest teilweise auf den Ueberhöhungen (oberes Niveau) oder zumindest teilweise auf beiden Niveaus. Dieser grobe Raster wirkt für die geführte Lichtwelle als Stopper der Wellenleitung.

Messungen, die im Scanmodus durchgeführt werden, sind weitgehend unempfindlich gegenüber kleinen Verkippungen und Verbiegungen, falls die Wellenleitergitterstruktur neben dem Sensorpfad einen Referenzpfad aufweist. In diesem Fall ist das referenzierte Sensorsignal die Differenz von Sensorsignal und Referenzsignal. Bei einer kleinen Verkippung der Wellenleitergitterstruktur bleibt diese Differenz (weitgehend) konstant.

Die Gitterperioden des (der) Einkopplungsgitter und des (der) Auskopplungsgitter können derart gewählt werden, dass Einkopplung und Auskopplung nur über die erste Beugungsordnung erfolgen. Es kann aber auch beim Einkopplungsgitter und/oder Auskopplungsgitter auch die zweite Beugungsordnung vorhanden sein. Die Gitterperioden von Einkopplungsgitter und Auskopplungsgitter sind gleich oder verschieden. Sind die Gitterperioden gleich, sind die Uerlegungen analog wie bei einem grossen Wellenleitergitter, wo es Gitterbereiche mit chemofunktionaler Signalschicht und Gitterbereiche (mit gleicher Gitterperiode) ohne chemofunktionale Signalschicht gibt. Bei Modenanregung im Wellenlängen-Scanmodus oder im Einfallswinkel-Scanmodus wird beim Einkopplungsgitter die Lichtverminderung und/oder beim Auskopplungsgitter die Lichterhöhung gemessen. Es kann aber auch sein, dass das beim Auskopplungsgitter ausgekoppelte Licht die Detektionsvorrichtung (Linse) nicht trifft. Das Auskopplungsgitter (mit gegenüber dem Einkopplungsgitter gleicher oder unterschiedlicher Gitterperiode) hilft Crosstalk zu vermindern. Vorteilhafterweise wird das Scanintervall (und die Gitterperioden) derart festgelegt, dass das Auskopplungsgitter während eines Scans nicht als Einkopplungsgitter wirkt. Aber selbst dieser Fall kann gehandhabt werden, da das nachfolgende Einkopplungsgitter (der nächsten Sensorstelle) dann einen ausgekoppelten Lichtstrahl (und damit Lichterhöhung) aufweist. Es sollte jedoch gewährleistet sein, dass es zwischen den benachbarten Sensorstellen nicht zur Resonanzüberlappung (Koinzidenz der beiden Resonanzscanparameter) kommt. Dies wird erreicht, indem die Gitterperioden von Einkopplungsgitter und Auskopplungsgitter entsprechend gewählt werden und/oder das Auskopplungsgitter mit keiner chemofunktionalen Signalschicht oder einer Passivierungsschicht belegt wird (Der Schichtdickenunterschied zwischen dem Bereich des Auskopplungsgitters einer Sensorstelle und dem Bereich des Einkopplungsgitters (mit chemofunktionaler Schicht) der nachfolgenden Sensorstelle soll derart gewählt sein, dass es zu keiner Resonanzüberlappung kommt).

Die nachfolgend beschriebenen Messungen im Wellenlängen-Scanmodus oder Einfallswinkel-Scanmodus sind auch mit einem (grossen) unidiffraktiven Gitter (oder mit mehreren unidiffraktiven Gittern) mit vier (oder weniger) einfallenden (aufgeweiteten) (gegebenenfalls unter 45 Grad gegenüber der Einfallsebene polarisierten) Lichtwellen zur Anregung der vier Moden TE+, TE-, TM+, TM- (vorwiegend im Grundmode m=0) möglich, wobei die Einfallswinkel derart eingestellt sein sollen, dass während eines Scanvorgangs die den Moden zugeordneten Resonanzkurven auf der Scanparameterachse nicht überlappen, d.h. um eine oder mehrere Resonanzbreiten jeweils voneinander getrennt sind. Ein möglicher Strahlengang schaut wie folgt aus: Der unter 45 Grad polarisierte Strahl fällt auf den Lichtstrahldeflektor (im Einfallswinkel-Scanmodus sicher vorhanden), anschliessend auf eine Strahlaufweitungsoptik und dann auf einen ersten Strahlteiler. Die beiden Teilstrahlen fallen je auf einen weiteren Strahlteiler, wobei dann je ein Teilstrahl auf den Sensor Chip und der andere Teilstrahl zuerst auf einen Spiegel und dann der reflektierte Strahl auf den Sensor Chip fällt. Es fallen also vier Teilstrahlen auf den Sensor Chip. Es ist aber auch möglich, Lichtstrahldeflektor und/oder Strahlaufweitung anstatt vor dem ersten Strahlteiler in vierfacher (gegebenenfalls zweifacher) Ausführung in den Strahlengang der vier Teilstrahlen zu setzen. Wird ein (grosse) bidiffraktives Gitter (Werden bidiffraktive Gitter) verwendet, genügen gegebenenfalls zwei unter 45 Grad polarisierte einfallende (aufgeweitete) Lichtstrahlen. Wird ein (grosses) multidiffraktives Gitter (Werden multidiffraktive Gitter) verwendet, genügt gegebenenfalls ein unter 45 Grad polarisierter einfallender (aufgeweiteter) Lichtstrahl. Es können aber immer auch vier einfallende (aufgeweitete) Lichtstrahlen eingesetzt werden.

Im Wellenlängen-Scanmodus mit Resonanzkurvenseparation oder im Einfallswinkel-Scanmodus mit Resonanzkurvenseparation ist die winkelmässige Separation der ausgekoppelten Lichstrahlen (siehe z.B. Figuren 3,4,5) unwesentlich.

Bei einem eindimensionalen Array von Wellenleitergitterstrukturen bzw. Sensorstellen müssen die Wellenleitergitterstrukturebene und die Detektorebene nicht parallel zueinander sein.

Die Scanmodi erlauben Messungen mit grossem dynamischen Bereich. Mit der Einführung einer Grobjustierung kann der dynamische Bereich sogar noch vergrössert werden (siehe weiter unten).

Bei den Scanmodi können Mittelungsverfahren zur Messignalbestimmung auch dann eingesetzt werden, wenn sich das Messignal während des Mittelungsverfahrens leicht verschiebt.

Im **Wellenlängen-Scanmodus (Typ I)** wird die Wellenlänge des (aufgeweiteten) einfallenden Lichtstrahls (Licht bezieht sich auf das sichtbare, infrarote und ultraviolette Spektrum) bei festem Einfallswinkel durchgestimmt. (Der einfallende Lichtstrahl soll möglichst als ebene Welle mit einem Wellenvektor bzw. als Gaussscher Strahl mit möglichst kleiner Divergenz vorliegen.) Gegebenenfalls kann der Einfallswinkel mit einem Lichtstrahldeflektor winkelmässig grob vorjustiert werden. Es kann jedoch auch (ohne Lichtstrahldeflektor) zuerst ein grosses Scanintervall vermessen werden (=Grobjustierung), um anschliessend darin ein kleineres Scanintervall, das die Resonanz enthält, festzulegen. Ist für ein (chemofunktionales) Einkopplungsgitter einer Wellenleitergitterstruktureinheit (bzw. Sensorstelle) bei einer bestimmten Wellenlänge λ (=Resonanzwellenlänge) die Einkopplungsbedingung N(λ)= n sin(α) +|I| (λ/∧) erfüllt (N(λ) = effektive Brechzahl des Modes bei Wellenlänge λ, α = Einkopplungswinkel, I = Beugungsordnung, A = Gitterperiode, n = Brechzahl der Luft), kommt es zur Modenanregung und die Intensität des ausgekoppelten bzw. abgestrahlten Lichts ändert sich resonanzförmig und kann mit einem Photodetektor (Photodetektorarray) in Funktion der Wellenlänge gemessen werden. Findet eine chemische Reaktion bzw. eine Massenanlagerung (z.B. infolge einer (bio)chemischen Bindung) mit effektiver Brechzahländerung ΔN statt, so verschiebt sich die Resonanzwellenlänge λ um Δλ zu der neuen Resonanzwellenlänge λ+Δλ. Für die Einkopplungsbedingung gilt nun N(λ+Δλ) + ΔN = n sin(α) + |I| ((λ+Δλ)/∧) = N(λ) + (|I|/∧)Δλ. Da in erster Ordnung N(λ+Δλ) = N(λ) + (dN(λ)/dλ)Δλ ist, gilt in erster Ordnung ΔN = (|I|/∧ - (dN(λ)/dλ))Δλ, wobei gemäss Modengleichung dN(λ)/dλ < 0 ist. Die Sensitivität dN(λ)/dλ hängt von der Polarisation und der Modenzahl des Modes ab, aber auch unter anderem von der Schichtdicke des wellenleitenden Films (von den Schichtdicken der Wellenleiterstruktur), von den Brechzahlen des Substrats, des wellenleitenden Films (der Wellenleiterschichtstruktur), des Covers und von der Brechzahl und Schichtdicke der chemofunktionalen Schicht ab. Für die Referenzierung mit Signalpfad und Referenzpfad gilt es zu beachten, dass die Beugungsordnung I (bzw. |I|) und dN(λ)/dλ auf beiden Pfaden gleich ist. Referenzierung mit einer dicken Schutzschicht (z.B. SiO2) auf dem Referenzpfad ist insofern mangelhaft, da sich dN(λ)/dλ ändert. Auch ist es wichtig, dass die chemofunktionale Signalschicht und die chemofunktionale Referenzschicht derselben Klasse angehören (siehe Abschnitt "Kompensation von Störungen") und/oder die beiden chemofunktionalen Schichten die gleiche (oder nahezu die gleiche) Brechzahl und/oder die gleiche (oder nahezu die gleiche) Schichtdicke aufweisen.

Vorzugsweise - aber nicht unbedingt - wird jene ausgekoppelte bzw. abgestrahlte Lichtwelle gemessen, welche sich nicht in Richtung des reflektierten Strahls ausbreitet. Dadurch kann der Background Noise reduziert werden.

Wird bei senkrechtem Lichteinfall (Einfallswinkel = 0 Grad) und in Richtung des reflektierten Lichtes gemessen, wird die ausgekoppelte bzw. abgestrahlte Lichtwelle über einen Strahlteiler geführt, bevor sie (über ein Linsensystem) auf den Detektor bzw. Detektorarray trifft. Dieser Strahlteiler (Rückseite der teildurchlässigen Schicht) kann auch als Strahlteiler zur Erzeugung des einfallenden Referenzstrahles dienen.

Zwischen Wellenleitergitterstruktur und Photodetektor kann sich ein Linsensystem befinden. Sind auf dem Sensor Chip mehrere Wellenleitergitterstruktureinheiten vorhanden, so werden die Intensitäten der verschiedenen ausgekoppelten bzw. abgestrahlten Lichtwellen mit einem ein(zwei)dimensionalen Array von Photodetektoren (mit oder ohne aufgesetztem Faserbündel) gemessen. Das gegebenenfalls zwischen Sensor Chip und Array von Photodetektoren befindliche Linsensystem kann ein eindimensionaler oder zweidimensionaler Array von Linsen (bzw. Linsensystemen) sein oder aber auch nur ein grosses Linsensystem (eine grosse Linse) sein.

Gitterperioden und/oder Orientierung der Gitter einer Wellenleitergitterstruktur und/oder Position des Linsensystems werden derart gewählt, dass je ein Photodetektor jeweils die Intensität einer ausgekoppelten bzw. abgestrahlten Lichtwelle misst. Der Photodetektor kann wiederum auch aus kleineren Einzeldetektoren bestehen, wobei aber immer nur die Intensität einer ausgekoppelten bzw. abgestrahlten Lichtwelle interessiert. Die kleineren Einzeldetektoren der verschiedenen Photodetektoren können zusammengefasst einen ein(zwei)dimensionalen Pixelarrayarray bilden.

Der (die) zwischen Sensor Chip und Detektor befindliche Linsenarray (sphärische oder zylindrische Linse) kann gegebenenfalls auch als Array von (Beinahe)Fourierlinsen wirken, da auch damit Intensitäten gemessen werden können, solange die Lichtfelder nicht überlagern. Die (Beinahe)Fourierebene bildet die Detektorebene oder kann über eine Linsenoptik auf den Detektor abgebildet werden.

Zwischen Sensor Chip und Detektor kann sich auch ein Faserbündel oder ein System von Faserbündeln (eventuell mit aufgebrachtem Linsensystem bzw. Linsenarray) befinden, das das abgestrahlte oder ausgekoppelte Licht oder das Streulicht der angeregten Moden misst. Vorteilhafterweise sind die Faserbündel in einer Reihe angeordnet.

Im Wellenlängen-Scanmodus (Typ I) kommt eine abstimmbare Lichtquelle (abstimmbarer Laser, abstimmbare Laserdiode, abstimmbarer VCSEL (vertical cavity surface emitting laser) (gegebenenfalls im Singlemode-Betrieb), Weisslichtquelle mit (durchstimmbarem) Monochromator (Prismenmonochromator, Gittermonochromator), Weisslichtquelle mit (durchstimmbarem) Fabry-Perot-Interferometer etc. zum Einsatz. Als abstimmbare Laserlichtquellen kommen gegebenenfalls Singlemode-Laserlichtquellen zum Einsatz. Die Lichtquellen können eine Kollimationsoptik beinhalten. Monochromatoren mit elektrooptischen (EO) oder akustooptischen (AO) Komponenten (wie z.B. EO oder AO Lichtstrahldeflektoren, EO oder AO Bragg-Gitter etc) sind Scanvorrichtungen ohne bewegliche Mechanik.

Bei Weisslichtquellen wird ein aufgeweiteter Lichtstrahl dadurch erzeugt, dass vorzugsweise zuerst die Lichtquelle auf eine Lochblende abgebildet wird und anschliessend über eine Linse aufgeweitet wird, wobei der Abstand Lochblende-Linse der Brennweite der Linse entspricht. Die Aufweitung kann eindimensional (unter Verwendung von (gekreuzten) Zylinderlinsen) oder zweidimensional (unter Verwendung von sphärischen Linsen oder gekreuzten Zylinderlinsen) erfolgen.

Bei Laserlichquellen mit kleiner Divergenz kommt zur Erzeugung eines (eindimensional oder zweidimensionl) aufgeweiteten Lichtstrahls ein Beam Expander (zwei sphärische Linsen unterschiedlicher Brennweite oder zwei Zylinderlinsen unterschiedlicher Brennweite oder zwei gekreuzte Paare aus je zwei Zylinderlinsen unterschiedlicher - Brennweite) zum Einsatz.

Vorteilhaft ist es, wenn der aufgeweitete Lichtstrahl möglichst gut eine ebene Welle simuliert, also weder konvergent noch divergent ist.

Die Lichtquellen können unpolarisiert oder polarisiert (linear, zirkular) sein. Durch Drehung eines linear polarisierten Lasers um die Laserachse kann z.B. 45 Grad linear polarisiertes Licht (bezüglich Einfallsebene) erzeugt werden. Linear polarisiertes Licht kann auch mit einem Polarisator erzeugt werden.

Es können mehrere Wellenlängenscans sehr schnell hintereinander durchgeführt werden und einem statistischen Mittelungsverfahren unterzogen werden. Aus einem Wellenlängenscan resultiert eine Resonanzkurve und daraus wird die Resonanzwellenlänge ermittelt. Diese Resonanzwellenlängen werden dann einem Mittelungsverfahren unterzogen. Typischerweise werden pro Sekunde oder pro Zeiteinheit 10 oder 100 oder 1000 etc Wellenlängenscans durchgeführt und einem Mittelungsverfahren unterzogen. Die Mittelungsverfahren erhöhen die Messgenauigkeit. Abstimmbare Laser oder abstimmbare Laserdioden oder VCSEL lassen eine rasche Durchstimmung (Scan) der Wellenlänge zu, da (falls) die Abstimmung ohne Einsatz von beweglicher Mechanik erfolgt.

Im einfallenden Lichtstrahl kann sich vor oder nach der Strahlaufweitung ein Beamsplitter (teildurchlässige Schicht, Glasplatte,Beugungsgitter etc) befinden, um auf einem gegebenenfalls mit einem Abschwächer versehenen 1 (2)-dimensionalen postions- und intensitätsempfindlichen Detektor (z.B. Pixelarraydetektor) (=Referenzdetektor) und gegebenenfalls unter Einsatz eines Beam Compressors oder Beam Expanders (oder einer Abbildungsoptik) das Beam Stirring und die Intensitätsschwankungen des einfallenden Lichtstrahls in Form eines Referenzsignals zu berücksichtigen. Ein Gitterbeamsplitter (in erster oder höherer Beugungsordnung) hat zudem den Vorteil, dass damit auf dem Referenzdetektor Wellenlängenschwankungen, die nicht dem eigentlichen Scanvorgang entsprechen, beobachtet werden können.

Beispielsweise kann das Beam Stirring über einen normalen Strahlteiler (z.B. Strahlteilerwürfel) und die Wellenlängenschwankungen (inklusive Beam Stirring) über einen Gitterbeamsplitter beobachtet werden. Dadurch lassen sich das Beam Stirring und die Schwankung der Wellenlänge voneinander separieren. Es können ein oder zwei Referenzdetektoren (mit einer oder zwei Beam Shaper Optik(en)) zum Einsatz kommen. Es können auch beide Lichtfelder nebeneinander auf einen Pixelarraydetektor fallen. Anstelle der Verwendung von einem normalen Strahlteiler und einem Gitterbeamsplitter kann bei einem einzigen Gitterbeamsplitter die nullte und eine höhere Beugungsordnung gemessen werden. Die nullte (reflektierte) Beugungsordnung übernimmt dabei die Funktion des normalen Strahlteilers (siehe auch Abschnitt "Einfallswinkel-Scanmodus")

Während eines Wellenlängenscans sollte es im Prinzip zu keiner Verschiebung Δ**x**(t) des (aufgeweiteten) Lichtstrahls (bzw. des Lichtschwerpunkts) in Funktion des Wellenlängenscanparamters t auf dem Referenzdetektor kommen. Es sollte also Δ**x**(t) = 0 sein.

Das Durchstimmen der Wellenlänge λ wird über die Aenderung des Wellenlängenscanparameters t erreicht. Die Wellenlänge λ(t) ist also eine Funktion (vielfach eine lineare Funktion) des Wellenlängenscanparameters t. Bei einem Wellenlängenscan durchläuft der Wellenlängenscanparamter t ein bestimmtes Intervall (tₛₜₐᵣₜ,t_{end}) und wird anschliessend zurückgefahren, sodass dann wiederum ein neuer Wellenlängenscan gestartet werden kann.

Tritt während den Scanvorgängen Beam Stirring auf, so ist zumindest zeitweise auf dem Referenzdetektor die Verschiebung Δ**x**(t) ungleich null, was einer winkelmässigen Verschiebung Δα(t) des festen Einfallswinkels α entspricht. Es gilt in erster Ordnung Ax(t) = L Δα(t), wobei im Fall eines normalen Strahlteilers L die Strahllänge zwischen Lichtquelle (Strahleinschnürung) und Referenzdetektor ist.

In einem Auswertungsverfahren wird das Beam Stirring während eines Scandurchgangs als konstant angenommen. Es kann das Beam Stirring vor und/oder nach einem Scandurchgang gemessen werden oder es kann aus mehreren während eines Scandurchgangs aufgrund von Beam Stirring verursachten Winkeländerungen Δα ein Mittelwert gebildet werden. Die Winkelstörung Δα verursacht eine Wellenlängenverschiebung (Störung) Δλ^{perturbation}, da wegen dN/dλ < 0 bei α+Δα die Einkopplungsgleichung N(λᵣₑₛ(α)+Δλ^{perturbation}) = n sin(α+Δα) + (I/∧)(λᵣₑₛ(α)+Δλ^{perturbation}) nur erfüllt werden kann, wenn sich auch die Wellenlänge λᵣₑₛ(α+Δα) = λᵣₑₛ(α)+Δλ^{perturbation} ändert. Aus der experimentellen Resonanzkurve, welche der Einkopplungsgleichung N(λᵣₑₛ(α)+Δλ^{perturbation}) = n sin(α+Δα) + (I/∧)(λᵣₑₛ(α)+Δλ^{perturbation}) entspricht, wird durch einen Fitalgorithmus (z.B. Schwerpunktsalgorithmus) die Resonanzwellenlänge λᵣₑₛ(α+Δα) = λᵣₑₛ(α)+Δλ^{perturbation} bestimmt. Mit N(λᵣₑₛ(α)+Δλ^{perturbation}) = N(λᵣₑₛ(α)) + (dN/dλ)Δλ^{perturbation} und N(λᵣₑₛ(α)) = n sin(a) + (I/∧)λᵣₑₛ(α) ergibt sich (dN/dλ - I/∧)Δλ^{perturbation} = n sin(α+Δα) - n sin(α). Ist N(λ) (bzw. dN/dλ) aus der Modengleichung oder aus einer Eichkurve bekannt, kann Δλ^{perturbation} und somit λᵣₑₛ(α) berechnet werden. Die korrigierte (auf den Einfallswinkel α bezogene) Resonanzwellenlänge ist somit λᵣₑₛ^{corr} = λᵣₑₛ(α+Δα) - Δλ^{perturbation} = λᵣₑₛ(α)

Neben dem Beam Stirring können auch Wellenlängenschwankungen Δλ^{perturbation} = Δλ^{wavelength shift} auftreten, die nicht dem eigentlichen Wellenlängenscanvorgang entsprechen. Die korrekte (korrigierte) Resonanzwellenlänge ist wiederum λᵣₑₛ^{corr} = λᵣₑₛ - Δλ^{perturbation}, wobei λᵣₑₛ die gemessene Resonanzwellenlänge darstellt. Kommen mehrere Störungen vor, so gilt λᵣₑₛ^{corr}= λᵣₑₛ - Σᵢ Δλ^{perturbation i}, wobei perturbation i die i-te Störung bedeuten soll.

In einem anderen Auswertungsverfahren bezüglich Kompensation von Beam Stirring wird mit einem Photodetektor (Photodetektorarray) die ausgekoppelte bzw. abgestrahlte Intensität in Funktion der Wellenlänge λ(t,α) = λ(t,α+Δα(t)) - Δλ^{perturbation}(t) gemessen (λ(t,α+Δα(t)) = λ(t)) und daraus mit einem Fitalgorithmus die korrigierte Resonanz λᵣₑₛ^{corrected} bestimmt, wobei die Störung Δλ^{perturbation}(t) via Δα(t)= Ax(t)/L und Gitterbeugungsgleichung des Einkopplungsgitters bestimmt wird. Anstelle von Beam Stirring Δα(t) soll die Störung gemäss Gitterbeugungsgleichung durch eine Wellenlängenänderung Δλ^{perturbation}(t) bewirkt werden, wobei in der Gitterbeugungsgleichung die Gitterperiode und die verwendete Beugungsordnung des Einkopplungsgitters einzusetzen ist. Es gilt sin(α+Δα(t)) = sin(α) +(I/∧)Δλ^{perturbation}(t), woraus sich Δλ^{perturbation}(t) in Abhängigkeit von Δα(t) berechnen lässt. Δ**x**(t) wird vom Referenzdetektor gemessen. Dieses Auswertungsverfahren kann ebenfalls auf mehrere Störungen erweitert werden, d.h es gilt λ(t,α) = λ(t,α+Δα(t)) - ΣᵢΔλ^{perturbation i}(t), wobei perturbation i die i-te Störung bedeuten soll. Auch ist es möglich, die Störung(en) während des Scanvorgangs als konstant anzunehmen, indem die Störung(en) vor und/oder nach dem Scanvorgang und/oder während des Scanvorgangs (als Mittelwert) gemessen werden. Zur Resonanzbestimmung wird die Intensität in Funktion von λ(t,α) ausgewertet.

Auch ein Startfehler im Wellenlängenscanparameter t (t durchläuft das Intervall (tₛₜₐᵣₜ,t_{end})) kann wie folgt kompensiert werden: Dadurch dass Sensorpfad und Referenzpfad gleichzeitig parallel gemessen werden, ist der Startfehler auf beiden Pfaden derselbe und kann deshalb wegreferenziert werden. Es ist λᵣₑₛ^{ref} = λᵣₑₛ^{signalpad} - λᵣₑₛ^{reference pad} das referenzierte (in Funktion der Zeit registrierte) Messignal, wobei λᵣₑₛ^{signal pad} die Resonanzwellenlänge für den Signalpfad und λᵣₑₛ^{reference pad} die Resonanzwellenlänge für den Referenzpfad darstellen.

Beim Arbeiten mit einem auf dem Sensor Chip befindlichen Referenzpfad kann gegebenenfalls auch auf die Bestimmung der korrigierten Wellenlängen λᵣₑₛ^{corr} verzichtet werden, solange die Störungen auf dem Signalpfad und dem Referenzpfad gleich sind und somit wegreferenziert werden können.

Im **Wellenlängen-Scanmodus (Typ II)** befindet sich das Scanelement im Gegensatz zum Wellenlängen-Scanmodus (Typ I) auf der Detektionsseite, d.h. im Strahlengang des von der Wellenleitergitterstruktur abgestrahlten bzw. ausgekoppelten Lichtfelds. Das Scanelement ist z.B. ein Monochromator oder ein Scanning-Fabry-Perot-Interferometer. Im Wellenlängen-Scanmodus (Typ II) kommt eine Weisslichtquelle (Breitbandlichtquelle) mit eindimensionaler oder zweidimensionaler Strahlaufweitung zum Einsatz. Die Wellenleitergitterstruktur wird unter festem Winkel beleuchtet. Gegebenenfalls kann (muss nicht) der einfallende Lichtstrahl winkelmässig mit einem Lichtstrahldeflektor grob vorjustiert werden.

Ueber einen Strahlteiler (Strahlteilerwürfel) und gegebenenfalls einen Beam Expander (oder Beam Compressor) kann auf einem zweiten Pixelarraydetektor (CCD, CCD-Kamera, Photodiodenarray, CMOS-Array, etc.) die Strahlqualität ((ortsaufgelöste) Intensitätsschwankungen, (winkelmässige) Strahlverschiebungen, etc.) (ortsaufgelöst) erfasst werden und mit dem Sensorsignal (ortsaufgelöst) referenziert werden. Zwischen Wellenleitergitterstruktur (mit oder ohne absorbierendem Wellenleiter) und Pixelarraydetektor befindet sich ein Linsensystem oder ein Array von Linsensystemen und ein Scanelement (mit gegebenenfalls dazugehöriger Optik). Wellenleitergitterstruktur und Detektionsfläche sind vorzugsweise parallel zueinander. Das Linsensystem kann als Abbildungslinse oder Beinaheabbildungslinse wirken. Das Linsensystem kann auch aus zwei Linsen bestehen und das Scanning-Fabry-Perot-Interferometer kann sich auch zwischen den beiden Linsen befinden.

Bei senkrechtem Lichteinfall wird das ausgekoppelte bzw. abgestrahlte Licht zuerst über einen Strahlteiler geführt. Die Rückseite dieses Strahlteilers kann als Strahlteiler für Erzeugung des einfallenden Referenzstrahls dienen.

Beim Durchstimmen des Scanelements in Funktion des Scanparamters t ändert die Transmissionswellenlänge des Scanelements. Entspricht die Transmissionswellenlänge der Resonanzwellenlänge, bei der bei einem bestimmten Sensorpfad (Signalpfad oder Referenzpfad) einer Wellenleitergitterstruktureinheit Modenanregung erfolgt, so ändert auf dem Pixelarraydetektor die Lichtverteilung an jenem Ort, der dem Sensorpfad entspricht. Die Aenderungen der Lichtverteilung (Lichtverminderung, Lichtintensivierung , Lichtschwerpunktsverschiebung innerhalb eines definierten Detektionsbereichs) in Funktion der Transmissionswellenlänge durchlaufen eine Resonanz. Die Auswertungen erfolgen wie beim Wellenlängen-Scanmodus (Typ I).

Im **Einfallswinkel-Scanmodus** wird der Einfallswinkel des aufgeweiteten einfallenden Lichtstrahls (Licht bezieht sich auf das sichbare, aber auch ultraviolette und infrarote Spektrum) mit Hilfe eines Lichtstrahl-Deflektors bei fester Wellenlänge durchgestimmt. (Der einfallende Lichtstrahl soll vorteilhafterweise als ebene Welle mit einem Wellenvektor (mit möglichst kleiner Divergenz) bzw. als Gaussscher Strahl mit möglichst kleiner Divergenz vorliegen.

Der Vorteil des Einkopplungswinkel-Scanmodus gegenüber dem Wellenlängen-Scanmodus mit abstimmbarer Laserlichtquelle ist die Verfügbarkeit von Laserlichtquellen fester Wellenlänge im grün/blauen, dunkelblauen und ultravioletten Wellenlängenbereich. Durchstimmbare Laserlichtquellen in diesem Wellenlängenbereich sind kostengünstig praktisch nicht verfügbar. Kürzere Wellenlängen erhöhen die Sensitivität der Wellenleitergittertstrukturen (siehe Thin Solid Films 126 (1985), 205-211). Zudem nimmt im kürzerwelligen Bereich die Lichtabsorption der Wellenleiter zu und muss gegebenenfalls nicht eingebaut werden.

Es können mechanische, galvanometrische, vibrierende, rotierende, elektrooptische (EO) oder akustooptische (AO) Lichtstrahl-Deflektoren (Scanner) zum Einsatz kommen. Mechanische Deflektoren sind z.B. rotierende oder vibrierende Spiegel, Polygonspiegel oder Keilplatten. Gegebenenfalls kann das durchzustimmende Einfallswinkelintervall winkelmässig mit einem zweiten Lichtstrahl-Deflektor grob vorjustiert werden, wobei sich der zweite Lichtstrahldeflektor vor oder nach dem ersten Lichtstrahldeflektor befinden kann. Dieser zweite für die Grobjustierung zuständige Lichtstrahldeflektor darf durchaus mechanisch bewegte Komponenten enthalten. Die grobe Vorjustierung kann aber auch über eine Wellenlängenänderung erfolgen, wofür es eine abstimmbare Lichtquelle (z.B. abstimmbare Laserdiode, abstimmbarer VCSEL) braucht. Die Lichtstrahlaufweitung kann vor oder nach dem (den) Lichtstrahl-Deflektor(en) erfolgen. Die Wellenleitergitterstruktur wird nicht gedreht, sondern es streift der eindimensional oder zweidimensional aufgeweitete Lichtstrahl über den Array von Wellenleitergitterstruktureinheiten(en) bzw. über den Array von Sensorstellen hinweg und ändert dabei den Einfallswinkel. Während dieser winkelmässigen Durchstimmung bleibt die Wellenleitergitterstruktur immer beleuchtet.

Vorzugsweise wird der Lichtstrahl-Deflektor zwischen Lichtquelle (bzw. Lichtquelle mit Koilimationsoptik) und Strahlaufweitung in den Strahlengang eingefügt. Der Lichtstrahldeflektor bewirkt, dass der Lichtstrahl etwas schräg die Strahlaufweitung durchquert. Die Anordnung bewirkt, dass ein aufgeweiteter Lichtstrahl winkelmässig durchgestimmt werden kann. Im Fall eines Laserstrahls hat der Lichtstrahl eine Gaussverteilung.

Nicht mechanische Lichtstrahldeflektoren (wie EO und AO Deflektoren) haben den Vorteil, dass sie sehr schnell arbeiten, da zur Strahlablenkung keine bewegliche Mechanik eingesetzt wird, und deshalb bei den Messungen statistische Mittelungsverfahren eingesetzt werden können. Es können so zum Beispiel mehrere Scans sehr schnell hintereinander durchgeführt und aus den Resonanzkurven die Einkopplungswinkel (=Resonanzeinfallswinkel) berechnet werden. Aus diesen Einkopplungswinkeln kann dann ein Mittelwert ermittelt werden. Typischerweise werden 10, 100 oder 1000 etc Einkopplungswinkelbestimmungen pro Sensorpfad pro Sekunde oder pro Zeiteinheit durchgeführt und einem Mittelungsverfahren unterzogen. Der gemittelte Messwert bildet dann einen gewichteten Messpunkt. Ein Mittelungsverfahren erhöht die Messgenauigkeit. Es können auch zuerst die Resonanzkurven überlagert (gemittelt) werden und dann aus der gemittelten Resonanzkurve der Einkopplungswinkel bestimmt werden. Es können aber auch die an den Mittelungsverfahren teilnehmenden Einzelwerte fortlaufend aufgenommen werden. Die Menge (bzw. der Definitionsbereich) der an der Mittelung teilnehmenden Einzelwerte wird dann fortlaufend nachgeschoben (Beispiel: Der älteste Messwert fällt aus dem Definitionsbereich, dafür kommt der nächst neuere Messwert hinzu).

Ist für einen Sensorpfad bei einem bestimmten Einfallswinkel (=Resonanzeinfallswinkel) die Einkopplungsbedingung erfüllt, kommt es zur Modenanregung und die Intensität des ausgekoppelten bzw. abgestrahlten Lichts steigt resonanzförmig an (bzw. die Intensität der direkt gebeugten Beugungsordnung (z.B. in Reflexion) nimmt resonanzförmig ab) und kann mit einem Photodetektor (Photodetektorarray) in Funktion des Einfallswinkels gemessen werden. Die beiden Bereiche des Lichtanstiegs und des Lichtabfalls sind typischerweise um eine Kopplunglänge zueinander versetzt. Wird der Resonanzeinfallswinkel durchfahren, so zeigt die Intensität des ausgekoppelten Lichts eine Resonanzkurve mit Maximum und die Intensität der direkt gebeugten Beugungsordnung (in Reflexion) eine Resonanzkurve mit Minimum. Findet eine Massenanlagerung (z.B. infolge einer (bio)chemischen Bindung) oder eine (bio)chemische Reaktion statt, so verschiebt sich der Resonanzeinfallswinkel.

Bei der Aufnahme der Resonanzkurve(n) kann die Lichtverteilung des (aufgeweiteten) einfallenden und sich während eines Scans zeitlich auf der Wellenleitergitterstruktur ortsmässig verschiebenden Lichtstrahls (z.B. die (projizierte) Gaussverteilung) berücksichtigt werden. Vorteil des Einfallswinkel-Scanmodus ist es, dass die Sensitiviäten des Wellenleiters, die von der Wellenlänge abhängen, - im Gegensatz zum Wellenlängen-Scanmodus - nicht nachkorrigiert werden müssen, da die Wellenlänge konstant ist.

Vorzugsweise - aber nicht unbedingt - wird jene ausgekoppelte bzw. abgestrahlte Lichtwelle gemessen, welche sich nicht in Richtung des reflektierten Lichtstrahls ausbreitet, was eine Reduktion des Background-Noise bewirkt.

Wird bei senkrechtem Lichteinfall (Einfallswinkel = 0 Grad) und in Richtung des reflektierten Lichtes gemessen, wird die ausgekoppelte bzw. abgestrahlte Lichtwelle über einen Strahlteiler geführt, bevor sie (über ein Linsensystem) auf den Detektor bzw. Detektorarray trifft. Dieser Strahlteiler (Rückseite der teildurchlässigen Schicht) kann auch als Strahlteiler zur Erzeugung des einfallenden Referenzstrahles dienen.

Zwischen Wellenleitergitterstruktur und Photodetektor kann sich ein Linsensystem befinden. Sind auf dem Sensor Chip mehrere Wellenleitergitterstruktureinheiten vorhanden, so werden die Intensitäten der verschiedenen ausgekoppelten bzw. abgestrahlten Lichtwellen mit einem eindimensionalen oder zweidimensionalen Array von Photodetektoren (mit oder ohne aufgesetztem Faserbündel) gemessen. Das gegebenenfalls zwischen Sensor Chip und Array von Photodektoren befindliche Linsensystem kann ein eindimensionaler oder zweidimensionaler Array von Linsen (bzw. Linsensystemen) sein oder aber auch nur ein grosses Linsensystem (eine grosse Linse) sein.

Gitterperioden und/oder Orientierung der Gitter einer Wellenleitergitterstruktur und/oder Position des Linsensystems werden derart gewählt, dass je ein Photodetektor jeweils die Intensität einer ausgekoppelten bzw. abgestrahlten Lichtwelle misst. Der Photodetektor kann wiederum auch aus kleineren Einzeldetektoren bestehen, wobei aber immer nur die Intensität (oder Gesamtintensität) einer ausgekoppelten bzw. abgestrahlten Lichtwelle interessiert. Die kleinen Einzeldetektoren der verschiedenen Photodetektoren können zusammengefasst einen eindimensionalen oder zweidimensionalen Pixelarraydetektor (Photodiodenarray, CCD-Array, CMOS-Array etc) bilden.

Der (die) zwischen Sensor Chip und Detektor befindliche Linsenarray (sphärische oder zylindrische Linse) kann gegebenenfalls auch als Array von (Beinahe)Fourierlinsen (als Beinahe(Fourier)linse) wirken, da auch damit Intensitäten gemessen werden können, solange die Lichtfelder nicht überlagern. Die (Beinahe)Fourierebene bildet die Detektorebene oder kann über eine Linsenoptik auf den Detektor abgebildet werden.

Zwischen Sensor Chip und Detektor kann sich auch ein Faserbündel oder ein System von Faserbündeln (eventuell mit aufgebrachtem Linsensystem bzw. Linsenarray) befinden, das das abgestrahlte oder ausgekoppelte Licht oder das Streulicht der angeregten Moden misst. Vorteilhafterweise sind die Faserbündel in einer Reihe angeordnet.

Im Einfallswinkel-Scanmodus kommt eine monochromatische Lichtquelle (Laser mit konstanter Wellenlänge, Laserdiode mit konstanter Wellenlänge, VCSEL mit konstanter Wellenlänge, Weisslichtquelle mit Wellenlängenfilter, Weisslichtquelle mit Monochromator (z.B. Gittermonochromator, Prismenmonochromator), Weisslichtquelle mit Fabry-Perot-Interferometer) zum Einsatz. Die Laser bzw. Laserdioden werden vorzugsweise im 'single mode' betrieben. Die Lichtquellen können eine Kollimationsoptik beinhalten. Laser bzw. Laserdioden können auch frequenzstabilisiert sein. Eine Laserdiode basiert ebenfalls auf dem Lasereffekt (d.h. es findet induzierte Emission statt).

Bei Weisslichtquellen wird ein aufgeweiteter Lichtstrahl dadurch erzeugt, dass vorzugsweise zuerst die Lichtquelle auf eine Lochblende abgebildet wird und anschliessend über eine Linse aufgeweitet wird, wobei der Abstand Lochblende-Linse der Brennweite der Linse entspricht. Die Aufweitung kann eindimensional (unter Verwendung von Zylinderlinsen) oder zweidimensional (unter Verwendung von sphärischen Linsen oder gekreuzten Zylinderlinsen) erfolgen.

Bei Laserlichquellen mit kleiner Divergenz kommt zur Erzeugung eines (eindimensional oder zweidimensionl) aufgeweiteten Lichtstrahls ein Beam Expander (zwei sphärische Linsen unterschiedlicher Brennweite oder zwei Zylinderlinsen unterschiedlicher Brennweite oder zwei gekreuzte Paare aus je zwei Zylinderlinsen unterschiedlicher Brennweite) zum Einsatz.

Vorteilhaft ist es, wenn der aufgeweitete Lichtstrahl möglichst gut eine ebene Welle simuliert, also weder konvergent noch divergent ist.

Im einfallenden Lichtstrahl kann sich vor oder nach der Strahlaufweitung und/oder vor oder nach dem Lichtstrahldeflektor ein Beamsplitter (teildurchlässige Schicht, Glasplatte, Beugungsgitter etc) befinden, um auf einem gegebenenfalls mit einem Abschwächer versehenen eindimensionalen oder zweidimensionalen positions- und intensitätsempfindlichen Detektor (z.B. Pixelarraydetektor) (=Referenzdetektor) das Beam Stirring und/oder die Intensitätsschwankungen des einfallenden Lichtstrahls in Form eines Referenzsignals zu berücksichtigen. Zwischen Strahlteiler (Beamsplitter) und Referenzdetektor kann sich ein Beam Expander oder Beam Compressor (=Anti-Expander) befinden, um den Strahldurchmesser an den Referenzdetektor anzupassen.

Ein Gitterbeamsplitter hat zudem den Vorteil, dass damit auf dem Referenzdetektor neben Beam Stirring und/oder Intensitätsschwankung des einfallenden Lichtstahls auch eine Wellenlängenschwankung der Lichtquelle beobachtet und das Messignal entsprechend korrigiert werden kann. In diesem Fall wird auf dem Referenzdetektor die plus/minus erste oder eine höhere Beugungsordnung beobachtet.

Vorteilhafterweise - aber nicht unbedingt notwendig - wird (werden) der (die) Beamsplitter zwischen Lichtquelle (gegebenenfalls mit Kollimationsoptik) und Lichtstrahldeflektor in den Strahlengang eingefügt.

Werden ein normaler Beamsplitter (z.B. Strahlteilerwürfel, teildurchlässiger Spiegel) und ein Gitterbeamsplitter unter Einsatz von einem oder zwei gegebenenfalls mit Beam Shaper Optik(en) und Abschwächer ausgestatteten Referenzdetektoren (z.B. ein(zwei)dimensionale PSD, ein(zwei)dimensionale Pixelarraydetektoren) hintereinandergeschaltet, so kann über den normalen Beamsplitter das Beam Stirring und über den Gitterbeamsplitter die Kombination von Beam Stirring und Wellenlängenschwankung mit dem (den) Referenzdetektor(en) beobachtet werden. Daraus lassen sich Beam Stirring und Wellenlängenschwankung separat bestimmen. Wird nämlich das über den normalen ersten Strahlteiler (z.B. Strahlteilerwürfel) gemessene erste Verschiebungssignal Δ**x₁** (= Beam Stirring) vom zweiten über den Gitterstrahlteiler (zweiter Strahlteiler) gemessenen Verschiebungssignal Δ**x₂** (= Beam Stirring und Wellenlängenschwankung) abgezogen, so repräsentiert das zweite mit dem ersten Verschiebungssignal referenzierte Verschiebungssignal Δ**x₂** - Δ**x₁** die Wellenlängenschwankung (Notation: Fette x bedeuten Vektoren). Vorzugsweise stehen die Gitterlinien des Gitterstrahlteilers senkrecht zur Einfallsebene und die Zeile eines Pixelarraydetektors parallel zur Einfallsebene. Die Referenzdetektoren werden vorzugsweise senkrecht bestrahlt. Schiefe Beleuchtung ist ebenfalls möglich, indem eine Verschiebung Δ**x** auf die Ebene senkrecht zum auf den Referenzdetektor auftreffenden Strahl projiziert wird. In diesem Fall ist in den nachfolgenden Rechnungen nicht Δ**x** sondern das projizierte Δ**x** zu berücksichtigen.

Anstelle von der Verwendung von zwei Strahlteilern kann bei einem Gitterstrahlteiler die nullte und eine höhere Beugungsordnung vermessen werden. Die nullte (reflektierte) Beugungsordnung übernimmt dabei die Funktion des normalen Strahlteilers (z.B. Strahlteilerwürfels). Es können zwei Referenzdetektoren zum Einsatz kommen, aber es können auch die nullte und die höhere Beugungsordnung separiert (gegebenenfalls unter Einsatz geeigneter Ablenkungsoptiken (z.B. Spiegeln) nebeneinander auf nur einen Referenzdetektor fallen. Es können z.B. auch die nullte (reflektierte) und eine höhere (die erste reflektierte) Beugungsordnung den gleichen Beam Expander (Compressor) unter verschiedenen Winkeln durchqueren.

Mitunter reicht es zu Kontrollzwecken auch aus, nur die höhere Beugungsordnung zu beobachten, da damit der additive Fehler aus Beam Stirring und Wellenlängenschwankung gemessen wird. Die Fehler können sich jedoch aufheben. In einer groben Approximation wird das mit einem Referenzdetektor gemessene Verschiebungssignal als Winkeländerung interpretiert.

Es können auch zwei normale Strahlteiler zum Einsatz kommen. Damit können neben dem einfallenden Lichtstrahl zwei Teilstrahlen generiert werden. Mit einem (ersten) Teilstrahl wird das Beam Stirring gemessen. Der andere (zweite)Teilstrahl fällt auf ein (Scanning)-Fabry-Perot-Interferometer (mit Optik) (in Transmission und/oder Reflexion) und anschliessend auf einen Detektor (Intensitätsdetektor und/oder positionsempfindlicher Detektor). Mit diesem anderen Teilstrahl wird also die Wellenlänge(nschwankung) (und das Beam Stirring) gemessen. Das Beam Stirring kann gegebenenfalls wiederum wegreferenziert werden. Es kann aber auch die Wellenlängenschwankung und das Beam Stirring als eine Grösse aufgefasst werden. In diesem Fall braucht es nicht den ersten Teilstrahl. Die Konfiguration kann auch im Wellenlängen-Scanmodus eingesetzt werden, indem z.B. vor oder nach einem Scandurchgang gemessen wird.

Das Durchstimmen des Einfallswinkels α wird über die Aenderung des Einfallswinkelscanparameter t (z.B. elektrisches Signal bei einem elektrooptischen Lichtstrahldeflektor) erreicht. Der Einfallswinkel α(t) ist also eine Funktion des Einfallswinkeiscanparameters t. Bei einem Einfallswinkelscan durchläuft der Einfallswinkelscanparamter t ein bestimmtes Intervall (tₛₜₐᵣₜ,t_{end}) und wird anschliessend zurückgefahren, sodass dann wiederum ein neuer Scan gestartet werden kann.

Im Fall eines Gitterbeamsplitters (betrieben in erster oder höherer Beugungsordnung) bewirkt eine Wellenlängenschwankung während eines Scanvorgangs eine Verschiebung Δ**x**(t) der Lichtverteilung auf dem Referenzdetektor in Funktion des Einfallswinkelscanparameters t. Unter der Annahme, dass kein Beam Stirring vorkommt, kann aus der Verschiebung Δ**x**(t) der Lichtverteilung auf dem Referenzdetektor unter Auswertung der Gitterbeugungsgleichung bezüglich des Gitterbeamsplitters die Wellenlängenänderung Δλ(t) ermittelt werden (Die Winkeländerung ist in erster Ordnung A**x**/L, wobei L der Strahlabstand der ersten oder höheren Beugungsordnung zwischen Gitterstrahlteiler und Referenzdetektor ist. Es kann jedoch auch exakt gerechnet werden).

Der Einfachheit halber werden die nachfolgenden Ueberlegungen für den Fall gemacht, dass sich der Gitterbeamsplitter zwischen Lichtquelle und Lichtstrahl-Deflektor befindet. Die Ueberlegungen gelten jedoch auch für den Fall, dass sich der Gitterbeamsplitter zwischen Lichtstrahl-Deflektor und Wellenleitergitterstruktur befindet. Mit der Verschiebung der Lichtverteilung auf dem Referenzdetektor ist in diesem Fall die Verschiebung der Lichtverteilung gemeint, die nicht dem eigentlichen Einfallswinkelscanvorgang entspricht. Es ist also die Abweichung von der Sollverschiebung gemeint.

In einem Auswertungsverfahren wird die Wellenlängenschwankung während eines Scandurchgangs als konstant angenommen. Es kann die Wellenlängenschwankung vor und/oder nach einem Scandurchgang gemessen werden oder es können die während eines Scanvorgangs gemessenen Wellenlängenschwankungen zuerst gemittelt werden. Die Störung Δλ der Wellenlänge λ verursacht eine Störung Δα^{perturbation} des Einkopplungswinkels, da wegen dN/dλ < 0 bei λ+Δλ die Einkopplungsgleichung N(λ+Δλ) = n sin(αᵣₑₛ(λ+Δλ))+ (I/∧)(λ+Δλ) nur erfüllt werden kann, wenn sich auch der Einkopplungswinkel αᵣₑₛ(λ+Δλ) = αᵣₑₛ(λ)+Δα^{perturbation} ändert. Aus der experimentellen Resonanzkurve, welche der Einkopplunsgleichung N(λ+Δλ) = n sin(αᵣₑₛ(λ+Δλ))+ (I/∧)(λ+Δλ) entspricht, wird durch einen Fitalgorithmus (z.B. Schwerpunktsalgorithmus) der bei λ+Δλ vorherrschende Resonanzeinfallswinkel αᵣₑₛ(λ+Δλ) = αᵣₑₛ(λ)+Δα^{perturbation} bestimmt. Mit N(λ+Δλ) = N(λ) + (dN/dλ)Δλ und N(λ) = n sin(αᵣₑₛ(λ))+ (I/∧)λ ergibt sich (dN/dλ - I/∧)Δλ = n sin(αᵣₑₛ(λ+Δλ)) - n sin(αᵣₑₛ(λ)). Ist N(λ) (bzw. dN/dλ) aus der Modengleichung oder aus einer Eichkurve bekannt, kann αᵣₑₛ(λ) bzw. Δα^{perturbation} berechnet werden. Der korrigierte (auf die Wellenlänge λ bezogene) Einkopplungswinkel (=Resonanzeinfallswinkel) ist demnach αᵣₑₛ^{corr} = αᵣₑₛ(λ+Δλ) - Δα^{perturbation} = αᵣₑₛ(λ).

Neben der Wellenlängenschwankung können auch noch Beam Stirring Δα^{perturbation} = Δα^{beam stirring} vorkommen. Der korrigierte Resonanzeinfallswinkel ist wiederum αᵣₑₛ^{corr} = αᵣₑₛ - Δα^{perturbation}, wobei αᵣₑₛ der gemessene Resonanzeinfallswinkel darstellt. Kommen mehrere Störungen vor, so gilt αᵣₑₛ^{corr} = αᵣₑₛ - ΣᵢΔα^{perturbation i}, wobei perturbation i die i-te Störung bedeuten soll.

In einem anderen Auswertungsverfahren bezüglich Kompensation von Wellenlängenschwankung wird mit einem Photodetektor (Photodetektorarray) die ausgekoppelte bzw. abgestrahlte Intensität in Funktion des Einfallswinkels α(t,λ) = α(t,λ+Δλ(t)) - Δ^{αperturbation} (t) gemessen (α(t,λ+Δλ(t)) = α(t)) und daraus mit einem Fitalgorithmus die korrigierte Resonanz αᵣₑₛ^{corrected} bestimmt, wobei die Störung Δα^{perturbation} (t) via Δλ(t) und Gitterbeugungsgleichung des Einkopplungsgitters bestimmt wird. Anstelle von Wellenlängenschwankung Δλ(t) soll die Störung gemäss Gitterbeugungsgleichung durch eine Winkeländerung Δα^{perturbation} (t) bewirkt werden, wobei in der Gitterbeugungsgleichung die Gitterperiode und die verwendete Beugungsordnung des Einkopplungsgitters einzusetzen ist. Es gilt sin(α(t,λ)+Δα^{perturbation} (t)) = sin(α(t,λ)) + (I/∧)Δλ(t), woraus sich Δα^{perturbation} (t) in Abhängigkeit von Δλ(t) berechnen lässt. Δλ(t) wird über Δ**x**(t) des Referenzdetektors und über die Gitterbeugungsgleichung des Gitterbeamsplitters bestimmt. Dieses Auswertungsverfahren kann ebenfalls auf mehrere Störungen erweitert werden, d.h. es gilt α(t,λ) = α(t,λ+Δλ(t)) - ΣᵢΔα^{perturbation i} (t), wobei perturbation i die i-te Störung bedeuten soll. Auch ist es möglich, die Störung(en) während des Scanvorgangs als konstant anzunehmen, indem die Störung(en) vor und/oder nach dem Scanvorgang und/oder während des Scanvorgangs (als Mittelwert) gemessen werden. Zur Resonanzbestimmung wird die Intensität in Funktion von α(t,λ) ausgewertet.

Ein Startfehler im Einfallswinkelscanparameter t kann - wie schon beim Wellenlängenscanmodus erwähnt - über den Referenzpfad auf dem Sensor Chip wegreferenziert werden. Beim Arbeiten mit einem auf dem Sensor Chip befindlichen Referenzpfad kann gegebenenfalls auch auf die Bestimmung von αᵣₑₛ^{corr} verzichtet werden, solange die Störungen auf dem Signalpfad und dem Referenzpfad gleich sind und damit wegreferenziert werden können.

Im **Auskopplungswinkel-Scanmodus** wird die winkelmässige bzw. auf dem Detektor örtliche Verschiebung (und/oder Intensitätsänderung) des (der) ausgekoppelten (monochromatischen) Lichtstrahlen mit Hilfe eines eindimensionalen oder zweidimensionalen Arrays von (positionsempfindlichen) Detektoren (z.B. PSD, PSD-Array, CCD, CCD-Kamera, Photodiodenarray, CMOS-Array etc.) gemessen, wobei sich vorzugsweise zwischen Wellenleitergitterstruktureinheit und Detektor eine (sphärische und/oder zylindrische) Linse oder Linsensystem befindet. Die monochromatische Lichtquelle ist vorzugsweise ein Laser, eine Laserdiode, ein VCSEL etc.. Die Laserlichtquellen werden bevorzugt monomodig und/oder frequenzstabilisiert betrieben.

Da in den Messvorgang keine bewegliche Mechanik involviert ist, eignet sich der Auskopplungswinkel-Scanmodus besonders gut zur Durchführung von statistischen Mittelungsverfahren.

Im einfallenden Lichtstrahlengang kann sich wiederum eine Beamsplitteranordnung (mit ensprechendem(n) Referenzdetektor(en)) befinden. Diese Beamsplitteranordnung kann z.B. aus der Kombination von einem normalen Beamsplitter (z.B. Strahlteilerwürfel) und einem Gitterbeamsplitter oder einem Gitterbeamsplitter betrieben in nullter und höherer Beugungsordnung bestehen (siehe auch Abschnitt Einkopplungswinkel-Scanmodus). Aus den Verschiebungen der beiden Lichtflecke auf dem (den) Referenzdetektor(en) kann eine Wellenlängenschwankung trotz eventuell gleichzeitig vorhandenem Beam Stirring bestimmt werden. Diese Wellenlängenschwankung kann bei der Auswertung der Verschiebung der ausgekoppelten Lichtstrahlen mitberücksichtigt werden. Infolge des Reziprozitätstheorems der Optik sind die Ueberlegungen beim Gittereinkoppler dieselben wie beim Gitterauskoppler.

Das Einkopplungsgitter (bzw. die Einkopplungsgitter der Wellenleitergitterstruktur bestehend aus mindestens einer Wellenleitergitterstruktureinheit) wird (werden) mit einer (eindimensional oder zweidimensional aufgeweiteten) ebenen oder (eindimensional oder zweidimensional aufgeweiteten) leicht fokussierten Welle(n) (Lichtkeilband oder ein(zwei)dimensionaler Array von Lichtkeilen gleichen oder unterschiedlichen Fokussierungsgrades) beleuchtet.

Gegebenenfalls ist die leicht fokussierte einfallende Lichtwelle winkelmässig mit folgender Vorrichtung einstellbar: Der Lichtstrahl wird mit einer Zylinderlinse auf einen Drehspiegel (beinahe) fokussiert und von dort mit einer zweiten Zylinderlinse auf das (die) Einkopplungsgitter fokussiert oder auch nur beinahe fokussiert. Durch Drehung des Drehspiegels ändert sich auch der Einfallswinkel des auf die Wellenleitergitterstruktur einfallenden konvergenten Lichtstrahls. Der Lichtstrahl kann zuerst (d.h. bevor er auf die erste Zylinderlinse trifft) auch über einen (einfachen oder zweifachen)(zylindrischen) Beam Expander zu einem eindimensionalen oder zweidimensionalen Lichtband geformt werden. Die Bandlänge ist parallel zur Drehachse des Drehspiegels.

Eine eindimensionale Lichtaufweitung erfolgt durch einen Beam Expander bestehend aus zwei Zylinderlinsen unterschiedlicher Brennweite. Eine zweidimensionale Lichtaufweitung erfolgt entweder durch einen Beam Expander bestehend aus zwei sphärischen Linsen unterschiedlicher Brennweite oder im Fall der Lichtaufweitung zu einem Lichtband durch einen ersten Beam Expander bestehend aus zwei Zylinderlinsen unterschiedlicher Brennweite und anschliessend durch einen zweiten Beam Expander bestehend aus zwei Zylinderlinsen unterschiedlicher Brennweite, wobei die Zylinderachsen des zweiten Beam Expanders senkrecht zu den Zylinderachsen des ersten Beam Expanders stehen.

Der aufgeweitete Lichtstrahl fällt auf eine Zylinderlinse (Zylinderachse ist senkrecht zur Einfallsebene), die den (leicht) fokussierten einfallenden Lichtstrahl (Lichtkeil) produziert. Der Fokus liegt auf der (oder in der Nähe) der Wellenleitergitterstruktur. Anstelle einer Zylinderlinse kann auch ein eindimensionaler oder zweidimensionaler Array von Zylinderlinsen (gleicher oder unterschiedlicher Brennweite) zum Einsatz kommen. Ein eindimensionaler Array von Zylinderlinsen (mit eventuell lichtdurchlässigem oder lichtundurchlässigem Zwischenabstand) produziert einen eindimensionalen Array von Lichtkeilen, welcher als (gerasterte) zweidimensionale Beleuchtung der Wellenleitergitterstrukur betrachtet werden kann. Die auf der Wellenleitergitterstruktur auftreffenden Lichtfelder (Lichtkeile) beleuchten die Einkopplungsgitter. Eine Zylinderlinse kann auch aus mehreren einzelnen Zylinderlinsen (mit eventuell lichtdurchlässigem oder lichtundurchlässigem Zwischenabstand) bestehen. Es entsteht so ein zweidimensionaler Array von Zylinderlinsen und damit ein zweidimensionaler Array von Lichtkeilen. Gegebenenfalls ist senkrecht zur Zylinderachse des ersten Zylinderlinsenarrays ein weiterer eindimensionaler oder zweidimensionaler Array von Zylinderlinsen vorhanden. Diese Zylinderlinsen sind dafür besorgt, dass die Lichtfelder (Lichtkeile) auch senkrecht zur Einfallsebene auf der Wellenleitergitterstruktur etwas gebündelt. Der Fokus dieser Zylinderlinsen kann oder kann auch nicht auf der Wellenleitergitterstruktur liegen. Anstelle der gekreuzten Arrays von Zylinderlinsen kann auch ein eindimensionaler oder zweidimensionaler Array von sphärischen Linsen (mit eventuell lichtdurchlässigem oder lichtundurchlässigem Zwischenabstand ) zum Einsatz kommen.

Im einfallenden Lichtstrahl kann sich vor und/oder zwischen und/oder nach den Beam Expandern zumindest ein Lichtstrahldeflektor befinden. Damit kann der Einfallswinkel des (eindimensional oder zweidimensional aufgeweiteten) einfallenden Lichtstrahls eingestellt werden. Es können auch zwei hintereinander geschaltete Lichtstrahldeflektoren zum Einsatz kommen, wobei ein Lichtstrahldeflektor für die Grobjustierung und ein Lichtstrahldeflektor für die Feinjustierung zuständig sind. Die Feinjustierung erfolgt zum Beispiel mit elektroopischen oder akustooptischen Deflektoren. Mit einem Lichtstrahldeflektor ist es auch möglich, den Einfallswinkel auch während der Messung zu ändern. Nach den Beam Expandern kann (muss aber nicht) das Linsensystem, das den Lichtkeil (die Lichtkeile) produziert, vorhanden sein.

Wird der einfallende Lichtstrahl über einen Strahlteiler geführt und werden die beiden Teilstrahlen anschliessend über je einen Spiegel auf die entsprechenden Einkopplungsgitter der Wellenleitergitterstruktur gelenkt, so kann mit einem Lichtstrahldeflektor, der sich vor dem Strahlteiler befindet, erreicht werden, dass die Einfallswinkel der beiden Teilstrahlen gleichzeitig betragsmässig grösser oder kleiner werden (siehe z.B. Figuren 5a, 5b, wobei die x-Komponenten der k-Vektoren (siehe Figur 2) sich addieren sollen)

Ist der Lichtstrahl zum Beispiel unter 45 Grad gegenüber der Einfallsebene linear polarisiert, so können in der Wellenleitergitterstruktur TE-Wellen als auch TM-Wellen (vorzugsweise im Grundmode) in Vorwärts- und/oder Rückwärtsrichtung angeregt werden.

Der Lichtstrahldeflektor (z.B. ein EO oder AO Deflektor) kann auch im Scanbetrieb eingesetzt werden. Beim Scanvorgang werden auf dem Detektor die Spots der ausgekoppelten Lichtfelder vorübergehend hell und erlauben zumindest zu diesem Zeitpunkt eine Ortsbestimmung der Spots.

Es können ein Mode im Signalpfad (mit chemofunktionaler Signalschicht) und ein Mode (gleicher oder unterschiedlicher Polarisation) im daneben liegenden Referenzpfad (mit chemofunktionaler Referenzschicht) angeregt werden. Bei unterschiedlicher Polarisation muss - falls die Sensitivität verschieden ist - die unterschiedliche Sensitivität bei der Referenzierung auf Signalebene berücksichtigt werden.

Es können aber auch ein Mode in Vorwärts- und Rückwärstrichtung oder der TE-Mode und TM-Mode in Vorwärts- und Rückwärstrichtung oder TE-Mode und TM-Mode in Vorwärtsrichtung oder TE-Mode in Vorwärtsrichtung (Rückwärtsrichtung) und TM-Mode in Vorwärts- und Rückwärtsrichtung oder TE-Mode in Vorwärts- und Rückwärtsrichtung und TM-Mode in Vorwärtsrichtung (Rückwärtsrichtung) bei geeigneter Wahl der Wellenleitergitterstruktureinheit angeregt werden. Die chemofunktionale Sensorschicht belegt zumindest teilweise die Auskopplungsgitter der Wellenleitergitterstrutureinheit oder auch die gesamte Wellenleitergitterstruktureinheit.

Zwischen Wellenleitergitterstruktur und posftionsempfindlichem Detektor (z.B. Pixelarraydetektor) befindet sich ein Linsensystem bzw. ein eindimensionaler oder zweidimensionler Array von Linsen bzw. Linsensystemen. Die Linsen können sphärische, zylindrische oder gekreuzt zylindrische Linsen sein.

Beispielsweise kann bei zwei gekreuzt zylindrischen Linsen eine Zylinderlinse (die Zylinderachse steht senkrecht zur Einfallsebene) für die Winkelmessung (d.h. die Zylinderlinse fokussiert beinahe auf den (Array von) (positionssensitiven) Detektor(en) (z.B. Pixelarraydetektor) bzw. eine Zwischendetektorebene) verwendet werden, während die andere Zylinderlinse den ausgekoppelten Lichstrahl senkrecht zur Einfallsebene etwas bündelt.

Vor dem Detektor kann sich auch zusätzlich eine (gekreuzt) zylindrische und/oder sphärische abbildende Linsenoptik befinden, die ein Zwischenbild (auf der Zwischendetektorebene) mit einer Linsenoptik (sphärisch und/oder (gekreuzt) zylindrisch) auf einen zweidimensionalen Pixelarraydetektor abbildet. Verschieben sich die Spots im Zwischenbild, so verschieben sie sich auch auf dem Pixelarraydetektor. Zwischen Wellenleitergitterstruktur und Detektor kann sich auch folgendes Linsensystem befinden: Eine erste Zylinderlinse (ein erster Array von eindimensional oder zweidimensional angeordneten Zylinderlinsen) mit Zylinderachsen senkrecht zur Einfallsebene wird in (Beinahe)-Fourierstellung betrieben. Gegebenenfalls hilft eine zweite zur ersten Zylinderlinse gekreuzte Zylinderlinse (die Zylinderachsen der ersten und zweiten Zylinderlinse stehen zueinander senkrecht) bzw. ein zweiter zum ersten Array gekreuzter Array von eindimensional oder zweidimensional angeordneten Zylinderlinsen den (die ausgekoppelten Lichtstrahl(en) senkrecht zur Einfallsebene etwas zu bündeln (oder zu fokussieren). Selbst wenn der zweite Array aus nur einer grossen Zylinderlinse besteht, gelingt es, die Lichtstrahlen etwas zu bündeln, ohne dass sie sich überlagern.

Die (Beinahe)-Fourierebene kann als fiktive Detektorebene (Zwischendetektorebene) aufgefasst werden. Diese fiktive Detektorebene (Zwischendetektorebene) wird mit zwei gekreuzten Zylinderlinsen(systemen) (gleicher oder unterschiedlicher Brennweite) (oder mit einem sphärischen Linsensystem oder mit einer Kombination von sphärischen und zylindrischen Linsensystemen) auf einen eindimensionalen oder zweidimensionalen Array von (positionssensitiven) Detektoren (z.B Pixelarraydetektor) abgebildet. Senkrecht zur Einfallsebene erfolgt die Abbildung über jene Zylinderlinse, deren Achse parallel zur Einfallsebene steht. Die dazu gekreuzte Zylinderlinse bildet die (aufgrund einer (bio)chemischen oder (bio)chemischen Reaktion verursachte) Verschiebung der (fiktiven) Lichtflecke der fiktiven Detektorebene (Zwischendetektorebene) auf die Detektorebene ab. Diese letzte Zylinderlinse kann (muss aber nicht) ein Vergrösserungverhältnis von eins aufweisen.

Die Kompensation von Störungen kann mit dem Konzept von Signalpfad und Referenzpfad erfolgen, wobei allerdings bestimmte - nachfolgend erwähnte - Voraussetzungen erfüllt sein sollten.

Zum Beispiel können Temperaturschwankungen und 'non specific binding' (NSB) mit dem Konzept von Signalpfad und Referenzpfad eliminiert werden. Ein Pfad besteht aus mindestens einem Gitter oder zumindest aus einem Einkopplungsgitter und einem Auskopplungsgitter. Die Temperaturschwankung und das NSB sollen auf dem Signalpfad und dem Referenzpfad gleich oder beinahe gleich sein. Auch die Sensitivitäten des Signalpfadsignals S(Signalpfad) (effektive Brechzahländerung, Kopplungswinkeländerung (beim Einfallswinkel-Scanmodus und/oder Auskopplungswinkel-Scanmodus), Wellenlängenänderung (beim Wellenlängen-Scanmodus), Intensitätsänderung, Aenderung der Intensität des Fluoreszenz (Lumineszenz, Elektrolumineszenz, Chemolumineszenz, Phosphoreszenz)signals) und des entsprechenden Referenzpfadsignals S(Referenzpfad) sollen gleich oder beinahe gleich sein. Dazu ist es notwendig (oder aber zumindest vorteilhaft), wenn die chemofunktionale Signalschicht (auf dem Signalpfad) und die chemofunktionale Referenzschicht (auf dem Referenzpfad) derselben Klasse von chemfunktionalen Schichten angehören. Folgende Klassen werden unterschieden: (1) Bindung bzw. Reaktion finden an der Oberfläche der chemofunktionalen Schicht statt (2) Bindung bzw. Reaktion finden im Volumen der chemofunktionalen Schicht statt (3) Binding bzw. Reaktion finden sowohl im Volumen als auch an der Oberfläche der chemofunktionalen Schicht statt.

Das Störsignal ΔS (z.B. effektive Brechzahländerung ΔN) ist in erster Ordnung die dem Störeffekt ΔX zugeordnete Sensitivität ∂S/∂X (z.B. ∂N/∂X) multipliziert mit dem Störeffekt ΔX. Bei gleicher Sensitivität und gleichem Störeffekt auf dem Signalpfad und Referenzpfad gilt S(Signalpfad) + ΔS(Signalpfad) - S(Referenzpfad) - ΔS(Referenzpfad) = S(Signalpfad) - S(Referenzpfad) = referenziertes Sensorsignal. Der Störeffekt wird wegreferenziert. Das Signal S wird meistens selbst als Signaländerung ΔS aufgefasst. Zwei wichtige Störungen sind die Temperaturschwankung und die nicht spezifische Bindung (NSB). Es gibt aber auch noch andere Störungen, wie Wellenlängenschwankung, Beam Stirring, Diffusion von Molekülen (Atome, Ionen) in die Wellenleiterstruktur, Quellung der chemofunktionalen Schichten, thermo-optische und photochemische Effekte etc..

Bei einer Temperaturschwankung ΔT(=Störeffekt) auf beiden Pfaden ist die relevante Sensitivität ∂N/∂T = (∂N/∂n_{c})(∂n_{c}/∂T), wobei n_{c} die Brechzahl des Covermediums C ist. Die anderen Beiträge - wie z.B. (∂N/∂n_{F})(∂n_{F}/∂T), wobei n_{F} die Brechzahl des wellenleitenden Films F ist - werden weggelassen, da sie bei der Referenzierung (mehr oder weniger) herausfallen. Die Referenzierung des Störsignals ist ΔN(S)-ΔN(R)=((∂N/∂n_{c})(S)(∂n_{c}/∂T)(S)-(∂N/∂n_{c})(R)(∂n_{c}/∂T)(R)) ΔT, wobei S=Signalpfad und R=Referenzpfad bedeuten. Gleiche Sensitivität ∂N/∂n_{c} und gleicher Temperaturkoeffizient ∂n_{c}/∂T auf beiden Pfaden ermöglichen, dass bei der Referenzierung das Störsignal herausfällt. Ist z.B. bei Bindung an die Oberfläche einer dünnen (z.B. monomolekularen, z.B. ca. 10 nm dicken) chemofunktionalen Signalschicht der Referenzpfad mit einer abschirmenden (Festkörper)Schicht (z.B. aus SiO2 belegt (die Abschirmung verhindert die Interaktion der evaneszenten Welle mit der Probe), so funktioniert die Referenzierung nicht, da der Temperaturkoeffizient ∂n_{c}/∂T auf dem Signalpfad (=Temperaturkoeffizient der flüssigen Probe) und der Temperaturkoeffizient ∂n_{c}/∂T auf dem Referenzpfad (=Temperaturkoeffizient des Festkörpers) unterschiedlich sind. Flüssigkeiten und Festkörper habe in etwa um eine Grössenordnung unterschiedliche Temperaturkoeffizienten.

Figur 9 zeigt die Sensitivät dN/dnC eines Wellenleiters bezüglich einer Brechzahländerung dnC des Covermediums C in Funktion der Dicke dF des wellenleitenden Films F. Die Sensitivätskurven hängen von der Schichdicke dA der chemosensitiven Schicht ab. Je grösser die Schichtdicke dA ist, desto kleiner wird die Sensitivität. Es bezeichnenTEO den transversal elektrischen Mode mit Modenzahl 0 und TMO den transversal magnetischen Mode mit Modenzahl 0.

Damit im Fall von Oberflächenflächenbindung die Sensitivitäten bezüglich einer Temperaturschwankung auf beiden Pfaden in etwa gleich sind, müssen die Schichdicke der chemfunktionalen Signalschicht und jene der chemofunktionalen Referenzschicht in etwa gleich sein. Läuft jedoch die evaneszente Welle in beiden Pfaden vollständig in der jeweiligen chemofunktionalen Schicht, so spielen die Schichdicken der chemofunktionalen Schichten keine Rolle.

Figur 10 zeigt die Sensitivät dN/d(dA) eines Wellenleiters bezüglich einer Schichtdickenänderung d(dA) der chemosensitiven Schicht A in Funktion der Dicke dF des wellenleitenden Films F. Die Sensitivätskurven hängen von der Schichdicke dA der chemosensitiven Schicht (A=adlayer) ab. Je grösser die Schichtdicke dA ist, desto kleiner wird die Sensitivität. Es bezeichnen TEO -den transversal elektrischen Mode mit Modenzahl 0 und TMO den transversal magnetischen Mode mit Modenzahl 0. Gleiche nicht spezifische Bindung (NSB) (gleiche Massenbelegung) an die Oberfläche einer dickeren chemosensitiven Schicht führt deshalb im Vergleich zu gleicher NSB (gleiche Massenbelegung) an eine dünnere chemosensitive Schicht zu einer kleineren effektiven Brechzahiänderung, da das Sensorsignal (z.B. Aenderung der effektiven Brechzahl) in erster Ordnung (linearer Fall) das Produkt aus Sensitivität und Störeffekt ist.

Findet die Bindung (bzw. die NSB) an die Oberfläche bei der chemofunktionalen Signalschicht und bei der chemofunktionalen Referenzschicht mit etwa gleicher Stärke (mit gleicher Belegungsdichte(Massenbelegung)) statt und sind beide chemofunktionalen Schichten gleich oder beinahe gleich dick, so sind die Sensitiväten (siehe Figur 10) der beiden Pfade gleich oder beinahe gleich und somit auch die Störsignale. Bei der Referenzierung des Signals (z.B. effektive Brechzahl(änderung), Einkopplungswinkel(änderung), Wellenlänge(nänderung) Auskopplungswinkel(änderung) etc.) werden demzufolge die Störsignale der beiden Pfade wegsubtrahiert.

Um generell Bindungen an der Oberfläche messen zu können, darf die Dicke dA der chemofunktionalen Schicht nicht zu gross sein (siehe Figur 10). Denn bei dickeren chemofunktionalen Schichten reduziert sich die Sensitivität dramatisch. Die Dicke der chemofunktionalen Schicht sollte kleiner als eine Wellenlänge, oder unter 100 nm liegen. Bevorzugt werden monomolekulare Schichten oder 'self-assembled monolayers' (mit gegebenenfalls darauf aufbauenden (bio)chemischen Strukturen).

Vorteilhaft ist es auch, Signalpfad und Referenzpfad gleichzeitig und in etwa mit der gleichen Intensität zu beleuchten. Dadurch werden in etwa auf beiden Pfaden die gleichen photochemischen und thermo-optischen Störungen ausgelöst und können deshalb wegreferenziert bzw. wegsubtrahiert werden. Thermo-optische Effekte in Kombination mit Wellenleitergitterstrukturen sind in Thin Solid Films 126 (1985), 197-203 beschrieben. Grundsätzlich ist es auch möglich, um die photochemischen und thermo-optischen Störungen gering zu halten, (a) mit geringen Lichtintensitäten und/oder (b) im Pulsbetrieb zu arbeiten. Andererseits muss natürlich die Lichtintensität genügend hoch sein, um ein Messignal zu erzeugen, das klar über dem Rauschen liegt.

Werden an einer Sensorstelle (Wellenleitergitterstruktur mit chemosensitiver Schicht) die Moden TE und TM in Vorwärts- und Rückwärtsrichtung (Modenzahl vorzugsweise gleich null) mit einem Scanmodus gemessen, so können ebenfalls mit Auswertungsverfahren, wie sie in WO 99/13320 beschrieben sind - jedoch vorzugsweise unter Verwendung von Wellenleitergitterstrukturen mit Strahlseparation, Sensorsignale mit reduzierter Temperaturabhängigkeit generiert werden. Beispielsweise kann das Sensorsignal die Schichtdicke des wellenleitenden Films (im Dreischichtmodell) oder die Schichdicke der Zusatzschicht (mit oder ohne (bio)chemosensitiver Schicht) sein und aus einem linearen Gleichungssystem mit dem Sensorsignal und der Temperaturschwankung ΔTals Unbekannte berechnet werden. Vielfach wird der Temperaturkoeffizient ∂N()/∂T (= Ableitung der effektiven Brechzahl N nach der Temperatur T bezogen auf einen Modetyp (0=TE oder 0=TM) als Inputgrösse verwendet.

Die Ueberlegungen zur Strahlführung des einfallenden Lichtstrahls (der einfallenden Lichtstrahlen), zur Modenanregung, zur Strahlaufweitung und zu den Lichtstrahldeflektoren können auf alle Scanmodi - also auch auf den Wellenlängenscanmodus oder auf den Einfallswinkelscanmodus - angewendet werden, wobei beim Wellenlängenscanmodus oder Einfallswinkelscanmodus (vorzugsweise) keine einfallenden Lichtkeile zur Anwendung kommen. Die Modenanregung erfolgt jedoch im Scanverfahren. Der Detektionsmechanismus in Kombination mit dem entsprechenden Scanmechanismus ist beim jeweiligen Scanmodus beschrieben. Das zwischen Wellenleitergitterstruktur und Detektor befindliche Linsensystem kann in Abbildungsstellung (oder Beinaheabbildungsstellung) und/oder Fourierstellung (oder Beinahe-Fourierstellung) betrieben werden.

Die Wellenleitergitterstrukturen (mit oder ohne Referenzierung), wie sie z.B. in den Figuren 2a, 2b, 2c beschrieben sind, können auch im Wellenlängen-Scanmodus oder Einfallswinkel-Scanmodus ausgelesen werden (Einkopplungsgitter und Auskopplungsgitter für einen Mode können zusammengenommen auch nur ein Gitter bilden). Während des Scanvorgangs kommt es bei allen vier Gittern zu Modenanregung. Die einfallenden Lichtstrahlen sind vorzugsweise unter 45 Grad gegenüber der Einfallsebene polarisiert, um sowohl TE-Wellen als auch TM-Wellen anregen zu können. Auf dem Pixelarraydetektor wird ortsaufgelöst entweder die bei Modenanregung resonanzförmig auftretende Lichtabschwächung und/oder (versetzt auftretende) Lichtintensivierung und/oder der (maximale) (absolute) Anstieg der Lichtverteilung zwischen der Lichtabschwächungsregion und der Lichtintensivierungsregion und/oder die Lichtschwerpunktsverschiebung der lichtverteilung während des Scans gemessen. Sind die Gitter überlagert, verliert man die Ortsauflösung. Die Gitterperioden müssen deshalb so gewählt werden, dass während eines Scanvorgangs die Resonanzcharakteristika zeitlich nacheinander auftreten. Der Wellenleiter kann (muss nicht) (gegebenenfalls lateral abhängig) absorbierend sein.

Die chemoempfindlichen Schichten können beispielsweise mit einem Spotter aufgebracht werden. Bei Streulichtmessungen und/oder Fluoreszenzmessungen können sich auch mehrere (verschiedene) chemoempfindliche Schichten arrayförmig ein(zwei)dimensional angeordnet zwischen zwei Gitter oder auf einem ausgedehnten Gitter befinden (eindimensionale Anordnung parallel oder senkrecht zu den Gitterstrichen oder zweidimensionale Anordnung). Streulicht, Lumineszenzlicht, Fluoreszenzlicht, Phosphoreszenzlicht kann mit einem Faserbündel (mit oder ohne aufgebrachtem Linsenarray) oder mit einem Linsensystem oder einem Linsenarray einem Detektor bzw. Detektorarray zugeführt werden.

Ein eindimensionaler Array von (verschiedenen) chemofunktionalen Schichten zwischen zwei Gittern (gleicher oder unterschiedlicher Gitterperiode) kann auch bei monochromatischen interferometrischen Direktmessungen (markierungsfreien Messungen) zum Einsatz kommen (vergleiche R.G. Heideman et al., Development of an Optical Waveguide Interferometric lmmunosensor, Proceedings Eurosensor 4, Karlsruhe 1990). Zu jeder Signalschicht eines eindimensionalen parallel zu den Gitterstrichen verlaufenden chemofunktionalen Signalschichtarrays gehört ein Referenzpfad (mit oder ohne in Modenrichtung gleich grossen chemofunktionalen Referenzschichten) oder zu mehreren (oder allen) eindimensional angeordneten Signalschichten gehört ein Referenzpfad (mit oder ohne in Modenrichtung gleich grosser chemofunktionaler Referenzschicht). Passivierungsmaterial, das NSB verhindet, belegt gegebenenfalls den Sensor Chip ausserhalb der chemofunktionalen Schichten. Das Einkopplungsgitter kann gerastert aus mehreren Einzeigittern (mit Zwischenabstand) bestehen, wobei die Einzelgitter jeweils das Licht für eine Signalschicht (bzw. Referenzschicht) besorgen. Bei einem durchgehenden Gitter kann das Gitter im Bereich des Zwischenabstandes mit einer Coverschicht belegt sein, um Lichteinkopplung nur ausserhalb der Coverschicht zu ermöglichen. Das Einkopplungsgitter (die Einzelgitter) können mit einem Lichtband oder Lichtbandkeil beleuchtet werden. Das Auskopplungsgitter kann gegebenenfalls gerastert aus mehreren Einzeigittern (mit oder ohne Zwischenabstand) bestehen. Gitterperioden, Gitterfokussierung, Gitterorientierung der Einzelgitter und gegebenenfalls der zwischen Auskopplungsgitter und Detektorarray (Pixelarraydetektor) befindliche Linsenrray sind derart ausgelegt, dass die jeweiligen Mach-Zehnder-Arme auf dem ein(zwei)dimensionalen Detektorarray (z.B. Pixelarraydetektor) interferieren. Die Detektorarrayebene kann auch eine Zwischenebene sein, die dann auf eine Detektorebene sphärisch, zylindrisch oder gekreuzt zylindrisch (mit unterschiedlichen Brennweiten) abgebildet wird. Die beschriebene Wellenleitergitterstruktur kann ein(zwei)dimensional arrayförmig erweitert werden. Das einfallende Licht sind mehrere Lichtbänder oder Lichtbandkeile oder ein aufgeweiteter Lichtstrahl. Die Herstellung des einfallenden Lichts ist weiter oben beschrieben. Es kann aber mit nur einem zweidimensionalen Detektorarray (z.B. Pixelarraydetektor) gearbeitet werden.

Das Gitter kann auch moduliert sein, wobei durchaus ortsaufgelöst ein Gitterbereich mit Modulationsstärke = 0 vorkommen kann und bedeutet, dass keine Gitterstriche vorhanden sind. Der ein(zwei)dimensionale Array von chemoempfindlichen Schichten kann z.B. in jenem Bereich liegen, der keine Gitterstriche aufweist. Auch muss die Gitterperiode nicht konstant sein.

Befindet sich im Strahlengang der abgestrahlten Lichtwelle(n) ein Strahlteiler, so kann gleichzeitig in einem (ersten) Strahlengang (mit Wellenlängenfilter, um Licht der Anregungswellenlänge abzublocken) Fluoreszenz(Lumineszenz, Phosphoreszenz)licht und im anderen Strahlengang gleichzeitig oder nicht gleichzeitig ein 'direct binding assay' gemessen werden. Im anderen (zweiten) Strahlengang kann sich auch ein Wellenlängenfilter befinden, das das Fluoreszenz(Lumineszenz, Phosphoreszenz)licht abblockt. Das (die) Linsensystem(e) kann (können) sich vor und/oder nach dem Strahlteiler befinden. Beispielsweise kann ein(e) sich zwischen Sensor Chip und Strahlteiler befindliche(s) Linse (Linsensystem) für den ersten Strahlengang (Pfad für Markierungsmessung) als Abbildungslinse und für den zweiten Strahlengang (Pfad für markierungsfreie Messung) als (Beinahe)Fourierlinse(nsystem) wirken. Die Linse (das Linsensystem) kann aber auch für beide Strahlengänge abbildend wirken (z.B. beim Einfallswinkel-Scanmodus oder Wellenlängen-Scanmodus mit kombinierter Fluoreszenz (Lumineszenz, Phosphoreszenz)Messung). Die Messungen auf dem markierungsfreien Pfad können auch als Referenzierung (z.B. Referenzierung der Intensität der Anregungswelle bei Fluoreszenz (Lumineszenz, Phosphoreszenz) messungen) dienen. Diese Referenzierung funktioniert auch, wenn parallel dazu, eine markierungsfreie Messung durchgeführt wird. Das Molekulargewicht des Labels (der Label) muss in bestimmten Assay-Formaten berücksichtigt werden.

Bei einem Einfallswinkelscan (oder Wellenlängenscan) durchläuft die in den zweiten Strahlengang (Pfad für markierungsfreie Messung) abgestrahlte (ausgekoppelte) Welle eine Resonanzkurve. Das in den ersten Strahlengang (Pfad für Markierungsmessung) abgestrahlte Fluoreszenz(Lumineszenz, Phosphorsezenz)licht durchläuft ebenfalls eine Resonanzkurve, welche nun mit der Resonanzkurve des ersten Strahlengangs referenziert werden kann. Dadurch, dass für die Referenzierung eine Resonanzkurve herangezogen wird, erhöht sich die Genauigkeit.

Es kann aber auch bei festem Einfallswinkel gearbeitet werden und die Linse (das Linsensystem) kann abbildend für beide Strahlengänge wirken. Bei Fluoreszenz (Lumineszenz, Phosphoreszenz) lichtmessungen dient die mit dem im zweiten Strahlengang befindlichen Detektor (Detektorarray) gemessene Intensität bzw. Intensitätsverteilung als Referenz (Referenzintensitätsverteilung) für die mit dem im ersten Strahlengang befindlichen Detekor(Detektorarray) gemessene Fiuoreszenz(Lumineszenz,Phosphoreszenz)lichtintensität(sverteilung). Referenzierung bedeutet in diesem Fall vorzugsweise eine Verhältnisbildung (MarkierungsintensitätlAnregungsintensität). Die Anregungsintensität wird vorteilhafterweise über ausgekoppelte (abgestrahlte) Lichtwellen gemessen.

Bei Verwendung eines Faserbündels kann man sich auch vorstellen, dass Faserbündel und Linsen (Linsensystem(e)) austauschbar bzw. verschiebbar sind. Mit dem Faserbündel wird beispielsweise eine Markierungsmessung (Fluoresenz, Lumineszenz, Phosphoreszenz) und mit der Linse (dem(den) Linsensystem(en)) ein Einfallswinkel-Scanmodus durchgeführt. Das Faserbündel (mit oder ohne aufgesetztem Linsensystem oder Linsenarray) nimmt vorzugsweise das Licht (Fluoreszenz, Lumineszenz, Streulicht) vom nicht modulierten Teil der Wellenleitergitterstruktur auf. Zwischen Faserbündel und Detektor oder zwischen Sensor Chip und Faserbündel oder zwischen zwei Faserbündelteilen befindet sich das Wellenlängenfilter. Das Ende des Faserbündels kann gegebenenfalls mit einer Linse (einem Linsensystem oder Linsen(system)array) auch auf den Detektor (Detektorarray) abgebildet werden. Die Intensität der Anregungswelle wird referenziert, indem in den einfallenden Strahlengang ein Beam Splitter aufgestellt wird und mit einem Detektor (Detektorarray) die Intensität des vom Beam Splitter reflektierten Lichts gemessen wird. Es kann jedoch auch (eventuell zusätzlich) die Intensität des vom Sensor Chip reflektierten bzw. des (vor und/oder nach der (den) chemoempfindlichen Schicht(en) und/oder unter der (den) chemoempfindlichen Schicht(en)) gebeugten Anregungslichts zur Referenzierung mit einem Detektor (Einzeldetektor, ein(zwei)dimensionaler Detektorarray (z.B. Pixelarraydetektor) mit oder ohne vorgeschalteter sphärischer oder zylindrischer Abbildungsoptik und mit oder ohne Wellenlängenfilter gemessen werden.

Es ist auch möglich, ein grösseres (stärker) moduliertes Wellenleitergitter (mit oder ohne gedämpftem (absorbierendem) Wellenleiter) mit einem eindimensionalen oder zweidimensionalen Array von chemoempfindlichen Schichten zu versehen. Wegen der stärkeren Modulation ist die Resonanzeinkopplungskurve relativ breit. Der den Mode anregende einfallende Lichtstrahl kann während der Messung fixiert sein oder auch im Einfallswinkel-Scanmodus betrieben werden. Die Wellenlänge ist so gewählt, dass Fluoreszenz(Lumineszenz)anregung möglich ist. Die Oberfläche des Sensor Chips wird mit einer Linse (einem Linsensystem) auf einen Array von Detektoren bzw. auf die Endfläche eines Faserbündels abgebildet. Die Lichtverteilung des anderen Endes des Faserbündels wird dann mit oder ohne Linse (Linsensystem, Linsenarray) einem Array von Detektoren zugeführt. Zwischen Sensoroberfläche und Detektor befindet sich wiederum ein Wellenlängenfilter, das nur das Emissionslicht durchlässt. Im abgestrahlten Licht kann sich wiederum ein Strahlteiler befinden, der eine Referenzierung des Emissionslichts bezüglich des abgestrahlten und/oder ausgekoppelten Anregungslichts ( Anregungsstreulichts) über den zweiten Strahlengang (siehe weiter oben) mit gegebenenfalls Emissionslicht blockierendem Wellenlängenfilter zulässt.

Beim Einfallswinkelscan (oder Wellenlängenscan) durchläuft die Fluoreszenz einer jeden Sensorstelle ein Maximum, da die anregende evaneszente Welle bezüglich Intensität ebenfalls ein Maximum durchläuft. Die reflektierte oder gebeugte Lichtwelle kann über einen sphärischen oder zylindrischen Beam Compressor oder eine Abbildungsoptik mit oder ohne Strahlteiler mit oder ohne Wellenlängenfilter auf einen ein(zwei)dimensionalen Detektorarray (z.B. Pixelarraydetektor) fallen und dient als Referenz.

Die Eigenfluoreszenz einer Wellenleitergitterstruktur auf einem Kunststoffsubstrat kann klein gehalten werden, indem sich zwischen Kunststoffsubstrat und wellenleitendem Film eine anorganische Schicht befindet, deren Brechzahl tiefer als jene des wellenleitenden Films ist und deren Dicke vorzugsweise grösser als die Eindringtiefe der anregenden geführten Lichtwelle ist. Die Gitterstruktur kann in diesem Fall z.B. photolithographisch in die anorganische Schicht oder in den anorganischen wellenleitenden Film aufgebracht werden.

Eine andere Messtechnik beruht auf der Messung von Emissionslicht (Fluoreszenz-, Lumineszenz-, Phosphoreszenzlicht) an Wellenleiter(gitter)strukturen in Kombination mit einer Direktmessung. Dabei ist eine Schicht der Wellenleiterstruktur (bestehend aus ein oder mehreren Schichten) und/oder eine Schicht zwischen wellenleitendem Film und Substrat und/oder eine Schicht zwischen wellenleitendem Film und Cover (bzw. (bio)chemofunktionaler Schicht) und/oder eine Schicht auf der Unterseite des Substrats und/oder das Substrat selbst lichtemittierend (Fluoreszenz-, Lumineszenz-, Phosphoreszenzlicht) bei Anregung mit Licht (mit breitem und/oder schmalem Anregungsspektrum). Diese Schicht kann z.B. eine Polymerschicht (oder eine festkörperähnliche Schicht oder eine glasartige Schicht) mit (hoher) Eigenfluoreszenz oder mit eingelagerten Emissionslichtmolekülen (Fluoreszenz-, Lumineszenz-, Phosphoreszenzmoleküle) sein. Die Emissionslichtwellenlänge ist unterschiedlich zur Anregungswellenlänge. Die Messmethodik beruht auf einem Einfallswinkel-Scanmodus oder auf einem Wellenlängen-Scanmodus (mit abstimmbarer Lichtquelle), wobei sich im Strahlengang zwischen Sensor Chip und Detektor ein Wellenlängenfilter (abblocken für das Anregungslicht, transparent für das Emissionslicht) befindet. Zwischen Sensor Chip und Detektor kann sich auch ein Strahlteiler befinden, wobei dann das Wellenlängenfilter vorzugsweise im Strahlengang zwischen Strahlteiler und Detektor steht. Das Anregungslicht kann z.B. durch den Strahlteiler auf den Sensor Chip ((bio)chemofunktionale Wellenleitergitterstruktur) fallen. Das Anregungslicht kann auch schief von der Substratseite her oder schief von der Coverseite her auf den Sensor Chip fallen, wobei der Strahlteiler vorhanden oder auch nicht vorhanden sein kann. Das Emissionslicht kann auch (vorzugsweise) in einer Richtung gemessen werden, die nicht der Reflexionsrichtung des Anregungslichtstrahls entspricht. Der einfallende Lichtstrahl erzeugt bei Erfüllung der Einkopplungsgleichung eine geführte Lichtwelle, kann aber auch das Emissionslicht erzeugen, das durch den geführen Mode direkt oder indirekt (resonanzförmig) verstärkt wird und/oder schwerpunktsmässigverschoben wird. Das (abgestrahlte und/oder ausgekoppelte) Emissionslicht der Emissionschicht wird auf den Detektor abgebildet. Das ausgekoppelte Licht der Anregungswelle kann oder kann auch nicht auf die Abbildungslinse fallen. Das ausgekoppelte Emissionslicht kann oder kann auch nicht auf die Abbildungslinse fallen. Die Messmethodik stellt eine Kombination von Direktmessung mit Fluoreszenz (Lumineszenz, Phosphoreszenz) messung dar. Da beim Wellenlängen-Scanmodus die (Anregungs)wellenlänge geschoben wird, muss das Anregungsspektrum genügend breit sein. Mit dem Verfahren lassen sich auch lichtemittierende Modebeating-Muster (zwischen dem TE-Mode und dem TM-Mode) und lichtemittierende inferometrische Muster mit und ohne (unidiffraktive oder multidiffraktive) Gitter mit Scanbetrieb (Einfallswinkel-Scanmodus oder Wellenlängen-Scanmodus) oder ohne Scanbetrieb (Anregung der Moden TE und/oder TM mit ebenen oder leicht fokussierten Wellen) unter Einsatz eines im Fall von Interferenz von TE- und TM-Licht zwischen Sensor Chip und Detektor befindlichen Polarisators (z.B. 45°-Polarisators) und unter Einsatz des erwähnten Wellenlängenfilters zur Zeit der Modenanregung messen, wobei die Moden der Anregungswellenlänge via Gittereinkopplung erzeugt werden. Eine Bindungsreaktion (bzw. Massenanlagerung) oder chemische Reaktion (Aenderung der komplexen Brechzahl) an der (bio)chemofunktionalen Schicht, die sich auf und/oder neben dem Gitter befindet, ändert die Periode des interferenzmusters.

Grundsätzlich können für alle Messtechniken alle Detektoren (Detektorarrays, Pixelarraydetektoren) auch gekühlte Detektoren sein. Speziell bei lichtschwachen Signalen kommen gekühlte Detektoren zum Einsatz. Die Kühlung kann z.B. mit PeltierElementen erfolgen. Die Menge der Detektoren wird als Detektorvorrichtung bezeichnet.

Grundsätzlich können auch absorbierende bzw. stark absorbierende Wellenleiter bzw. wellenleitende Filme bzw. wellenleitende Materialien (mit oder ohne ortsabhängig lichtabsorbierender Strukturierung) zum Einsatz kommen. Der wellenleitende Film kann zum Beispiel aus Schwarzglas sein oder mit einem absorbierenden Material (Metall, Chrom, Gold, Silber, Aluminium, Zinn, Zink, Titan, Farbstoff, Silizium, gefärbtes Glas, gefärbtes Polymer, gefärbter Lack, Polymer-Metallverbindungen etc.) dotiert sein. Das absorbierende Material kann aber auch als dünne Schicht vorliegen. Diese dünne Schicht kann sich beim Interface Substrat/Film oder Film/chemosensitive Schicht oder Film/Cover oder innerhalb der Wellenleiterstruktur (im Fall von einem Mehrschichtsystem) befinden. Sieht die (bio)chemosensitive Schicht die Oxidoberfläche können einfache Methoden zur Immobilisierung angewendet werden. Der wellenleitende Film (oder zumindest eine Schicht der Welleneleiterstruktur) sollte einen hohen Realteil der komplexen Brechzahl aufweisen. Der wellenleitende Film kann zum Beispiel aus einem Tantaloxid (Ta2O5), Titanoxid (TiO2), Zirkonoxid (ZrO2), Hafniumoxid (HfO2), Nioboxid, Oxynitride, Si3N4 etc. bestehen. Das Schwarzglas kann zum Beispiel mit einer Sputtertechnik aufgebracht werden. Das Substrat kann Glas oder Kunststoff sein.

Ein absorbierender Wellenleiter weist eine stark verkürzte Kopplungslänge auf. Ein absorbierender Wellenleiter stellt ein dissipatives System dar, d.h. die Energie der geführten Lichtwelle wird an den Wellenleiter abgegeben. Bei einer Wellenleitergitterstruktur fehlt dann im Fall von Modenanregung diese Energie in den direkt abgestrahlten Beugungsordnungen.

Ist der Wellenleiter nicht absorbierend, kann zwischen den Gittern eine Schicht (z.B. gefärbte Polymerschicht, gefärbte Lackschicht, gefärbte Glasschicht, Metallschicht, Halbleiterschicht, Polymer-Metall-Verbindungen etc.) aufgebracht sein, die als Modestopper wirkt. Die Moleküle dieser Schicht können (müssen aber nicht) in den wellenleitenden Film eindringen. Das Aufbringen dieser den Mode stoppenden Schicht kann zum Beispiel mit einem Spotter erfolgen. Diese den Mode stoppende Schicht kann auch in Form eines Kreisrings vorliegen und gegebenenfalls eine chemosensitive Schicht umschliessen.

Absorbierende Wellenleiter, Auskopplungsgitter oder auch Stoppgitter, Stopptaper, Stopprillen (mit oder ohne Unterbruch des wellenleitenden Films) helfen, den Crosstalk zwischen zwei Sensorstellen zu vermindern.

Nichtabsorbierende Wellenleitergitter mit einem stark modulierten Gitter zeigen auch eine kurze Kopplungslänge. Die Energie der geführten Lichtwelle wird jedoch im Wellenleiter verheizt.

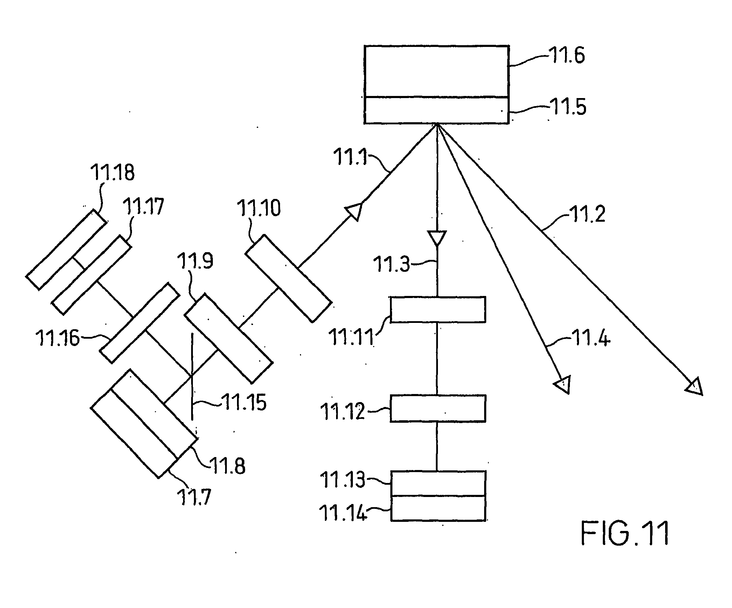

Figur 11 zeigt eine mögliche Messanordnung zur Parallelauslese von einem eindimensionalen und/oder zweidimensionalen Array von (chemofunktionalen) Wellenleitergitterstruktureinheiten in schematischer Weise.

Der einfallende Lichtstrahl 11.1 liegt als aufgeweiteter Lichtstrahl oder als Lichtstrahlbündel vor. Der reflektierte Strahl 11.2 läuft nicht in die Detektionsvorrichtung 11.11 bis 11.14. Das Lichtfeld 11.3 kann eine Beugungsordnung der Wellenleitergitterstruktur sein, kann aber auch nur ein Streulichtfeld (oder Fluoreszenzlichtfeld) sein. Ein weiteres Beugungsfeld 11.4 (falls vorhanden) tritt nicht in die Detektionsvorrichtung 11.11 bis 11.14 ein.

Die Wellenleitergitterstruktur 11.5 bestehend aus mindestens einer Wellenleitergitterstruktureinheit bzw. einer Sensorstelle ist gegebenenfalls mit einer Flüssigkeitshalterungsvorrichtung 11.6 bestehend aus einer Well oder einer Durchflussküvette oder einer Kapillarküvette oder aus einem Array von Wells und/oder Durchflussküvetten und/oder Kapillarküvetten.

Die Lichtquelle 11.7 erzeugt das Licht und ist gegebenfalls bereits mit einer ersten Optik (z.B. Kollimationsoptik) ausgestattet.

Das Linsensystem (oder Array von Linsensystemen) 11.10 erzeugt einen aufgeweiteten Lichtstrahl oder ein Lichtstrahlbündel (= Bündel von (aufgeweiteten und/oder (leicht) fokussierenden) Lichtstrahlen).

Das Linsensystem (oder Array von Linsensystemen) 11.11 lenkt ortsaufgelöst die abgestrahlten oder ausgekoppelten Lichtfelder auf den Detektor 11.13. Das Linsensystem (oder Array von Linsensystemen) 11.11 kann abbildend (oder beinahe abbildend), als Fourierlinse(n) wirkend (oder als beinahe Fourierlinse(n) wirkend oder fokussierend (oder leicht fokussierend) oder lichtsammelnd (oder leicht lichtsammelnd) operieren.

Der Detektor 11.13 besteht aus einem Array von Detektoren, der ortsaufgelöst Lichtintensitäten misst. Der Detektor 11.13 kann auch ein Pixelarraydetektor sein, welcher als spezieller Array von Detektoren aufgefasst werden kann. Der Detektor kann aber auch aus einem Array von positionssensitiven Detektoren bestehen.

Die Scanvorrichtungen 11.8 (Wellenlängenscanvorrichtung im auf die Wellenleitergitterstruktur-einstrahlenden Lichtfeld, Wellenlängenshifter),11.9 (Winkelscanvorrichtung, Lichtstrahldeflektor bzw. Lichtstrahldeflektorsystem für die Grob- und Feinjustierung), 11.12 (Wellenlängenscanvorrichtung im von der Wellenleitergitterstruktur abstrahlenden Lichtfeld), 11.14 (Auslesevorrichtung zum Auslesen des vom Detektor 11.13 gemessenen Lichtfelds bzw. der gemessenen intensitätsverteilung) beziehen sich auf unterschiedliche Messmodi, wobei die Auslesevorrichtung 11.14 des Detektors immer vorkommt.

Die Wellenlängenscanvorrichtung 11.8 gegebenenfalls inklusive Wellenlängenshifter zur Grobjustierung bezieht sich auf den Wellenlängen-Scanmodus (Typ 1). Der Lichtstrahldeflektor 11.9 muss nicht vorhanden sein, kann jedoch gegebenenfalls für die Grobjustierung (Festlegung eines vernünftigen Scanintervalls) eingesetzt werden. Die Wellenlängenscanvorrichtung 11.12 ist nicht vorhanden.

Die Wellenlängenscanvorrichtung 11.12 bezieht sich auf den Wellenlängen-Scanmodus (Typ II). Der Lichtstrahldeflektor 11.9 muss nicht vorhanden sein, kann jedoch gegebenenfalls für die Grobjustierung (Festlegung eines vernünftigen Scanintervalls) eingesetzt werden. Die Wellenlängenscanvorrichtung 11.8 ist nicht vorhanden (oder ist in Form eines Breitbandfilters zur Festlegung eines maximalen Messintervalls vorhanden).

Die Winkelscanvorrichtung 11.9 bezieht sich auf den Einfallswinkel-Scanmodus. Dabei ist die Wellenlängenscanvorrichtung 11.12 nicht vorhanden. Die Wellenlängenscanvorrichtung 11.8 muss nicht vorhanden sein, kann jedoch gegebenenfalls als Wellenlängenshifter für die Grobjustierung (Festlegung eines vernünftigen Scanintervalls) eingesetzt werden ( Achtung: Bei Wellenlängenänderungen ändern sich auch die Sensitivitäten der Wellenleitergitterstruktur (siehe und vergleiche Thin Solid Films 126 (1985), 205-211) und müssen (können) bei der Auswertung berücksichtigt werden. Die Grobjustierung kann aber auch mit einem zweiten Lichtstrahldeflektor vorgenommen werden.

Beim Auskopplungswinkel-Scanmodus braucht es auf jeden Fall auch die Auslesevorrichtung 11.14. Lichtstrahldeflektor 11.9 und/oder Wellenlängenshifter 11.8 können zur Grobjustierung herangezogen werden. Beim Wellenlängenshifter müssen gegebenenfalls die Sensitivitäten (siehe und vergleiche Thin Solid Films 126 (1985), 205-211 ) nachkorrigiert werden.

Bei Fluoreszenz(Lumineszenz)messungen kann 11.12 auch nur ein Wellenlängenfilter (ohne oder mit Scanvorrichtung) darstellen.

Oberhalb der Wellenteitergittersturktureinheit(en) kann sich eine Flüssigkeitshalterung oder Probenaufnahmevorrichtung oder eine Küvette (Wellküvette, Durchflussküvette, Kapillarküvette) oder ein Array von Flüssigkeitshalterungen und/oder Probeaufnahmevorrichtungen und/oder Küvetten und/oder Kombinationen davon befinden. Vorteilhafterweise sind die Flüssigkeitshalterungen, Probenaufnahmevorrichtungen und Küvetten dunkel (schwarz). Auch das Verklebematerial zwischen Sensor Chip und Küvette (Probenaufnahmevorrichtung, Flüssigkeitshalterung) ist vorzugsweise dunkel bzw. schwarz. Dunkle (schwarze) Materialien reduzieren das Streulicht und somit den Crosstalk.

Eine spezielle Flüssigkeitshalterung (Probenhalterung) kann ein an einer Nadel (injektionsnadel) oder Pipettenspitze hängender Flüssigkeitstropfen (Probentropfen) darstellen, der die Sensorstelle(n) kontaktiert, ohne dass die Nadel (Injektionsnadel) oder Pipettenspitze den Sensor Chip kontaktiert. Diese Situation kann zum Beipiel mit einem Abstandssensor kontrolliert werden. Durch geringes Ausstossen und Ansaugen der Flüssigkeit (Probe) kann eine Bewegung der Flüssigkeit (Probe) erreicht werden. Die Bewegung der Flüssigkeit (bzw. Probe) hilft, nicht spezifische Bindungen (NSB) zu unterdrücken. Ein Array von Injektionsnadeln oder Pipettenspitzen bringt die Proben oder die Probentropfen an den Array von (chemosensitiven) Wellenleiterstruktureinheiten bzw. Sensorstellen. Der Array von Probenzuführungsvorrichtungen (z. B. lnjektionsnadeln oder Pipettenspitzen) kann während der Messung via Proben mit dem Sensor Chip verbunden sein. Der Array von Probenzuführungsvorrichtungen und der Sensor Chip können gegebenenfalls (separate) Temperierungsvorrichtungen (mit oder ohne Einsatz von Peltierelementen) mit dazugehörigen Temperatursteuerungen aufweisen. Der Array von Probenzuführungsvorrichtungen (z.B. injektionsnadein oder Pipettenspitzen) kann aber auch nach dem Aufbringen der Proben bzw. Probentropfen auf den Sensor Chip komplett entfernt werden. Der Kontakt zwischen Sensor Chip und Probenzuführungsvorrichtung via Proben besteht also nur zeitweise. Die Probentropfen haften durch die Oberflächenspannungen (Oberflächenkräfte), welche somit sozusagen ein intrinsische Probenhalterungen darstellen. Die Wells können in den Sensor Chip eingebracht sein und nur geringe Vertiefungen aufweisen. Es können aber auch keine Wells vorhanden sein.

In eine Wellküvette kann ein Rührer eingefügt oder eine Pipettenspitze oder Injektionsnadel eingeführt werden. Mit dem Rührer kann die Flüssigkeit in Bewegung gehalten werden. Ueber eine Pipettenspitze oder injektionsnadel kann die Flüssigkeit (bzw. Probe) kontinuierlich zugefügt und abgesaugt werden. Die Absaugung kann auch über eine zweite Pipettenspitze oder Injektionsnadel erfolgen, womit ein Durchfluss entsteht. Auch damit entsteht eine Bewegung der Flüssigkeit (bzw. Probe). Die Bewegung der Flüssigkeit (bzw. Probe) hilft, nichtspezifische Bindung (NSB) zu minimieren. Bei der Durchflussküvette hat man automatisch mit dem Durchfluss eine Bewegung der Flüssigkeit (bzw. Probe). Bei der Kapillarküvette entsteht die Bewegung der Flüssigkeit (bzw. Probe) durch die Kapillarwirkung. Ist die Kapillare gefüllt, so kommt ohne weiteres Zutun die Bewegung der Flüssigkeit (bzw. Probe) zum Stillstand.

Die Flüssigkeitshalterung (Probenhalterung)oder Probenaufnamevorrichtung oder Küvette kann verschieden ausgelegt sein: Die in der Vorrichtung befindliche Probe (bzw. Flüssigkeit) kann z.B. nur eine chemofunktionale Signalschicht gegebenenfalls inklusive chemofunktionaler Referenzschicht kontaktieren, d.h. über jeder chemoempfindlichen Signalschicht gegebenenfalls inklusive chemofunktionaler Referenzschicht befindet sich eine Vorrichtung (Durchflusszelle, offene Küvette, Well, Kapülarküvette, etc). Es kann sich jedoch über der chemofunktionalen Referenzschicht auch eine separate Vorrichtung befinden.

Unter einer Vorrichtung können sich aber auch mehrere (oder gar alle) chemosensitiven Schichten befinden. In diesem Fall kontaktiert ein und dieselbe Probe mehrere (oder gar alle) chemosensitiven Schichten. Die Vorrichtung mit den mehreren chemosensitiven Schichten können matrixmässig oder kreisringmässig erweitert werden und so eine (übergeordnete) Array-Anordnung bilden. Die matrixmässige Erweiterung kann z.B. die Form einer Wellplate (mit z.B. 24, 48, 96, 384,1536 Wells etc ) oder eines Microarray haben. Unter jeder Vorrichtung (z.B Well) befindet sich dann eine (matrixförmige) Anordnung von chemosensitiven Schichten. Es kann sich jedoch unter jeder Vorrichtung gegebenenfalls auch nur eine chemosensitive Schicht befinden. Kontaktiert-die Probe die chemofunktionale Signalschicht und die chemofunktionale Referenzschicht in derselben Vorrichtung oder Probenhalterung ist die Temperaturgleichheit auf beiden Pfaden sehr gut gewährleistet.

Der Referenzpfad (mit oder ohne chemofunktionale Referenzschicht) kann auch nur mit Wasser oder einer Pufferlösung (vorteilhafterweise die Pufferlösung der Probe) bedeckt sein. Vorteilhafterweise ist - im Fall von Oberflächeninteraktion - die Dicke der chemofunktionalen Referenzschicht gleich oder beinahe gleich wie jene der chemofunktionalen Signalschicht, um Gleichheit der Sensitivität (zum Beispiel Temperatursensitivität) sicherzustellen. Da die Pufferlösung keine Biomoleküle enthält, kann auch keine NSB der Biomoleküle im Referenzpfad auftreten. Die chemofunktionate Signalschicht und die chemofunktionale Referenzschicht können hier (müssen nicht) identisch sein. In diesem Fall hat der Signalpfad und der Referenzpfad eine separate Flüssigkeitsaufnahmevorrichtung (Well, Durchflussküvette, Kapillarküvette, Flüssigkeitshalterung (Probenhalterung) etc).

Die Durchflusszellen können mit einem Zufuhrschlauch und Abfuhrschlauch versehen sein. Die Durchflussküvette (oder Kapillarküvette) kann aber auch mit zumindest einer Membran versehen sein, durch die die Nadel eines Liquid Handling Systems stechen und die Probe zuführen (bzw. abführen) kann. Ueber eine Dichtungsvorrichtung kann (können) aber auch eine (zwei) Pipettenspitze(n) oder Nadel(n) an die Durchflusszelle (oder Kapillarküvette) herangefahren werden und diese flüssigkeitsdicht auf der Zufuhrseite und/oder Abfuhrseite kontaktieren. Die Bewegung der Flüssigkeit (bzw. Probe) erfolgt durch Stossen der Flüssigkeit (bzw. Probe) von der Zufuhrseite her oder durch Saugen der Flüssigkeit (bzw. Probe) von der Abfuhrseite her.

Die Wells bzw. Durchflussküvetten bzw. Kapillarküvetten können als separate Probenzellen (bzw. Probenzellenplatte) auf die Wellenleitergitterstruktur bestehend aus ein oder mehreren Wellenleitergitterstruktureinheiten (Sensorstellen) aufgebracht werden. Es ist jedoch auch möglich, das Substrat derart mit Vertiefungen zu versehen, dass diese Vertiefungen bereits die Funktion der 'wells' bzw. der Durchflussküvetten bzw. der Kapillarküvetten übernehmen. In den letzen beiden Fällen muss die Wellenleitergitterstruktur mit einer mit Bohrungen versehenen Deckplatte abgedeckt werden.Im ersten Fall kann die Wellenleitergitterstruktur mit einer Deckplatte (ohne Bohrungen) abgedeckt werden, um zum Beispiel Verdunstung zu vermeiden. Die Bohrungen dienen der Zufuhr bzw. Abfuhr der Flüssigkeit (bzw. Probe) und/oder der Entlüftung.

Um die Empfindlichkeit pro Probenvolumeneinheit weiter zu steigern, ist es auch vorteilhaft, die Probe mehrfach über die Sensorstelle, bestehend aus mindestens einem sensing pad einer Wellenleitergitterstruktureinheit mit eventueller Referenzierung, laufen zu lassen. Dies kann z.B dadurch erreicht werden, dass die Probe im Fall einer Durchflussküvette abwechslungsweise vorwärts und rückwärts bewegt (gepumpt) wird. Eine andere Möglichkeit besteht darin, dass die Durchflussküvette Teil eines "sample loop" ist. Im sample loop hat es eine Flüssigkeitsbewegungsvorrichtung (z.B. Pumpe), die bewirkt, dass die im loop eingeschlossene Probenmenge mehrfach über die Sensorstelle kreisförmig in eine Richtung und/oder abwechselnd vorwärts und rückwärts bewegt wird. Der sample loop wird vorzugsweise über ein Ventil(system) gefüllt und geschlossen, wobei vorzugsweise Ventile mit möglichst kleinem Todvolumen - oder gar mit Todvolumen = 0 - zum Einsatz kommen. Der sample loop kann zumindest teilweise aus Schläuchen bestehen und/oder aber auch Teil eines lab-on-chip Systems sein. Vorteil eines lab-on-chip Systems ist es, dass Ventile, Bewegungsvorrichtung (z.B. Pumpe) und Flüssigkeitskanäle auf einem Chip (lab-on-chip) integriert sind. Die Sensorstelle kann sich auf einem separaten Sensor Chip befinden, die mit dem lab-on-chip in Kontakt gebracht wird, oder aber auch in den lab-on-chip integriert sein. Im letzteren Fall ist dann z.B, zumindest ein sensing pad (mit oder ohne Referenzierung) in eine Flüssigkeitskanalwand integriert. Die Flüssigke'rtskanalwand weist dann an ein oder mehreren Stellen parallel (an gegenüberliegenden Flüssigkeitswänden) oder seriell eine Wellenleitergitterstruktur mit vorzugsweise unterschiedlichen (bio)chemofunktionalen Signalschichten (und gegebenenfalls mit (bio)chemofunktionalen Referenzschichten) auf. Sensorstelle und Vorrichtung zur Flüssigkeitsbewegung (z.B. Pumpe) können im Flüssigkeitskanal seriell angeordnet sein oder auch parallel vortiegen (d.h. gegenüber liegen). Es können auch parallel mehrere Flüssigkeitskanäle vorhanden sein. Die Sensorstellen der Flüssigkeitskanäle können parallel oder seriell augelesen werden. Durch geeignetes Ventilsteuerungsdesign können die Flüssigkeitskanäle parallel mit der gleichen Probe oder parallel mit verschiedenen Proben angesteuert werden. Es können die Flüssigkeitskanäle auch einzeln angesteuert werden.

Die Sensibilisierung der Sensorstelle (Auftragen der (bio)chemofunktionalen Schicht) kann gegebenenfalls auch über ein zweites Flüssigkeitskanalsystem mit entsprechenden Ventilen erfolgen. Die Sensorstelle kann also durch ensprechende Schaltung der Ventile einmal Bestandteil des ersten Flüssigkeitskanalsystems und einmal Bestandteil des zweiten (oder gar dritten, vierten etc.) Flüssigkeitskanalsystems sein. Ueber das zweite Kanalysystem kann z.B. die Immobilisierung der (bio)chemoempfindlichen Schicht erfolgen.

Eine spezielle Wellenleitergitterstruktur bestehend aus einem (zwei) Einkopplungsgitter und einem (zwei) Auskopplungsgitter mit dazwischen liegender (bio)chemofunktionaler Signalschicht (und gegebenenfalls daneben liegender (bio)chemofunktionaler Referenzschicht) kann auch als Mach-Zehnder-Interferometer betrieben werden, wobei ein Mach-Zehnder-Arm über die (bio)chemofunktionale Signalschicht und der andere Mach-Zehnder-Arm über die (bio)chemofunktionale Referenzschicht führt (siehe R.G. Heideman et al., Development of an Optical Waveguide Interferometric lmmunosensor, Proceedings Eurosensor 4, Karlsruhe, 1990). Einkopplungsgitter und Auskopplungsgitter können gleiche oder unterschiedliche Gitterperioden aufweisen. Die (bio)chemofunktionale Referenzschicht soll keine (oder beinahe keine) NSB aufweisen oder die gleiche (oder beinahe gleiche) NSB aufweisen wie die (bio)chemofunktionale Signalschicht. Es gelten die gleichen wie schon oben ausgeführten Betrachtungen betreffend Sensitivität und Störeffekt. In unserem Fall kommen jedoch vorzugsweise transparente Substrate zum Einsatz, um Lichteinfall von der Substratseite her zu ermöglichen.

## Patentansprüche

1. Optischer Sensor zur Bestimmung mindestens einer chemischen oder biochemischen Substanz oder zum Studium des Zellverhaltens, aufweisend folgende Komponenten:
- eine Breitband-Lichtquelle (11.7) zur Erzeugung eines einfallenden Lichtfelds,
- einen Sensorchip mit einer Wellenleitergitterstruktur auf einem Substrat, wobei die Wellenleitergitterstruktur einen Array von Wellenleitergitterstruktureinheiten oder Sensorstellen aufweist,
- wobei die Wellenleitergitterstruktur mindestens teilweise mit mindestens einer chemosensitiven und/oder biochemosensitiven Substanz oder mit Zellen bedeckt ist;
- wobei die Wellenleitergitterstruktur mindestens einen Wellenleiter und mindestens ein Gitter aufweist und befähigt ist, mindestens ein Lichtfeld auszusenden, wenn das einfallende Lichtfeld die Wellenleitergitterstruktur mindestens teilweise beleuchtet,
- ein Scanelement oder Monochromator, und
- einen Detektor,
- wobei das Scanelement oder der Monochromator im Strahlengang zwischen der Wellenleitergitterstruktur und dem Detektor angeordnet ist.

2. Optischer Sensor nach Anspruch 1, **dadurch gekennzeichnet, dass** der Monochromator durchstimmbar ist.

3. Optischer Sensor nach Anspruch 1 oder 2, **dadurch gekennzeichnet, dass** sich die Transmissionswellenlänge des Scanelements in Funktion eines Scanparameters ändert.

4. Optischer Sensor nach einem der vorangehenden Ansprüche, **dadurch gekennzeichnet, dass** das Scanelement bzw. der Monochromator als ein PrismenMonochromator, ein Gitter-Monochromator oder ein Scanning-Fabry-Perot-Interferometer ausgebildet ist.

5. Optischer Sensor nach einem der vorangehenden Ansprüche, **dadurch gekennzeichnet, dass** der Detektor einen Array von Detektoren oder positionssensitiven Detektoren aufweist.

6. Optischer Sensor nach einem der vorangehenden Ansprüche, **gekennzeichnet durch** eine eindimensionale oder zweidimensionale Strahlaufweitungsoptik, die im Strahlengang zwischen der Lichtquelle und der Wellenleitergitterstruktur angeordnet ist.

7. Optischer Sensor nach einem der vorangehenden Ansprüche, **dadurch gekennzeichnet, dass** die Zellen lebende Zellen sind.

8. Optischer Sensor nach einem der vorangehenden Ansprüche, **dadurch gekennzeichnet, dass** die Zellen Pflanzenzellen oder Tierzellen oder Humanzellen sind.

9. Optischer Sensor nach einem der vorangehenden Ansprüche, **dadurch gekennzeichnet, dass** sich zwischen der Wellenleitergitterstruktur und den Zellen eine modifizierte Oberfläche oder eine biokompatible Oberfläche befindet.

10. Optischer Sensor nach einem der vorangehenden Ansprüche, **dadurch gekennzeichnet, dass** zumindest eine Flüssigkeitsaufnahmevorrichtung vorhanden ist.

11. Optischer Sensor nach einem der vorangehenden Ansprüche, **dadurch gekennzeichnet, dass** zumindest eine Wellenleitergitterstruktureinheit oder Sensorstelle einen Signalpfad und einen Referenzpfad aufweist.

12. Optischer Sensor nach Anspruch 11, **dadurch gekennzeichnet, dass** der Signalpfad und der Referenzpfad zur gleichen Flüssigkeitsaufnahmevorrichtung oder zu verschiedenen Flüssigkeitsaufnahmevorrichtungen gehören.

13. Verfahren zum Studium des Zellverhaltens, in welchem eine Zellen umfassende (bio)chemofunktionale Schicht auf eine Wellenleitergitterstruktur, beinhaltend mehrere Wellenleitergitterstruktureinheiten oder Sensorstellen, aufgebracht wird, wobei die Wellenleitergitterstruktur mindestens teilweise beleuchtet wird, wodurch das Aussenden mindestens eines Lichtfelds von der Wellenleitergitterstruktur bewirkt wird, und wobei das ausgesandte Lichtfeld gemessen und **dadurch** das Zellverhalten studiert wird.

14. Verfahren nach Anspruch 13, **dadurch gekennzeichnet, dass** zumindest eine Wellenleitergitterstruktureinheit oder Sensorstelle einen Signalpfad, der mit der (bio)chemofunktionalen Schicht belegt ist, und einen Referenzpfad aufweist, und dass aus einem vom Signalpfad erzeugten Signal und einem vom Referenzpfad erzeugten Signal ein referenziertes Sensorsignal gewonnen wird.

15. Optischer Sensor zum Studium des Zellverhaltens, aufweisend zumindest eine Wellenleitergitterstruktur auf einem Substrat, die Wellenleitergitterstruktur beinhaltend mehrere Wellenleitergitterstruktureinheiten oder Sensorstellen, wobei eine Wellenleitergitterstruktureinheit oder Sensorstelle zumindest einen Signalpfad aufweist, wobei der Signalpfad eine (bio)chemofunktionale Schicht umfasst, **dadurch gekennzeichnet, dass** die (bio)chemofunktionale Schicht Zellen umfasst.
